# EUROPEAN PATENT APPLICATION

(11) **EP 4 721 730 A1**
(43) Date of publication of application: **08.04.2026**
(21) Application number: 24815948.5
(22) Date of filing: 03.06.2024
(51) Int. Cl.: A61K 9/16, A61K 38/22, A61K 38/26, A61P 3/10, A61P 3/04, A61P 1/16, A61P 25/28

(54) **PHARMACEUTICAL COMPOSITION COMPRISING SUSTAINED-RELEASE MICROSPHERE COMPRISING CAGRILINTIDE OR PHARMACEUTICALLY ACCEPTABLE SALT THEREOF AND PREPARATION METHOD THEREFOR**

(30) Priority: 02.06.2023 KR 20230071360
(71) Applicant: G2GBIO, Inc., Cheongju-si, Chungcheongbuk-do 28161 (KR)
(72) Inventor: LEE, Heeyong, Daejeon 34032 (KR); LEE, Jinwoo, Sejong-si 30144 (KR); KIM, Hyemin, Cheongju-si Chungcheongbuk-do 28222 (KR); KIM, Boyeon, Cheongju-si Chungcheongbuk-do 28222 (KR); SEOL, Eunyoung, Daejeon 34080 (KR); BYEON, Hongju, Jeungpyeong-gun Chungcheongbuk-do 27927 (KR)
(74) Representative: Ricker, Mathias
(86) International application number: PCT/KR2024/007596
(87) International publication number: WO 2024/248574

(57) **Abstract**

The present invention relates to a pharmaceutical composition comprising sustained-release microspheres composed of cagrilintide or a pharmaceutically acceptable salt thereof, an initial burst inhibiting agent, and a biodegradable polymer, which has no rapid initial burst of the drug, contains a high content of the drug relative to the particle size, and has high bioavailability, thereby minimizing patient suffering and inflammatory responses that may occur upon administration to the human body, and is useful for the prevention or treatment of diabetes, beta-cell dysfunction, hypertension, hyperlipidemia, obesity, non-alcoholic steatohepatitis, or Alzheimer's disease.

## Description

### [Technical Field]

The present invention relates to a sustained-release microsphere including a high content of cagrilintide or a pharmaceutically acceptable salt thereof, a sustained-release microsphere including cagrilintide and semaglutide or pharmaceutically acceptable salts thereof at the same time, or a combination (blending) of the sustained-release microsphere including the cagrilintide or the pharmaceutically acceptable salt thereof and the sustained-release microsphere including the semaglutide or the pharmaceutically acceptable salt thereof, and a pharmaceutical composition including the microsphere or the microsphere combination, and a method of manufacturing the same.

### [Background Art]

Amylin is a 37-amino acid peptide produced in pancreatic beta cells and secreted together with insulin. The amylin significantly affects appetite, glucose management, and gastric emptying that warrant research for treatment of diabetes and obesity. Human amylin is also involved in cytotoxic amyloid formation observed in islet of Langerhans of a patient with type 2 diabetes. In 2005, a variant of amylin (pramilitide discovered by Amylin Pharmaceuticals) was approved for type 1 diabetes and type 2 diabetes along with insulin. The pramilitide includes three proline substitutions compared to the human amylin. The pramilitide has a short half-life (20 to 45 minutes) and is injected subcutaneously three times a day, which has a problem that an injection frequency is high. The pramilitide has consistently caused weight loss in several trials alone or in combination with leptin, but has side effects such as nausea and vomiting at a start of treatment.

Meanwhile, cagrilintide is a recently developed long-acting amylin analogue. It is also known that the cagrilintide may be combined with semaglutide that is a current glucagon-like peptide-1 (GLP-1) agonist to achieve sustained weight loss in people with amylin overweight and obesity. The amylin that is released together with the insulin from beta cells of a pancreas induces a satiety effect through a homeostatic region and a hedonic region of a brain. The semaglutide that is a GLP-1 receptor agonist decreases appetite through GLP-1 receptor of a hypothalamus, increases insulin production, and decreases glucagon secretion to delay gastric emptying. The amylin analogue and the GLP-1 receptor agonist are different like this, but a related mechanism of action appears to have an additional effect on appetite reduction. Considering heterogeneity and complex pathogenesis of obesity, a combination therapy using various pathophysiological targets is a logical approach to increase a weight loss response by pharmacotherapy, and the cagrilintide alone and in combination with the semaglutide showed promising weight loss in a clinical trial supporting further development of this therapy for sustained weight management.

However, for effective administration of the cagrilintide alone or in combination, development of a technology to ensure a long-term effective pharmacological effect is still required. Accordingly, the inventor of the present invention devises a technique for encapsulating the cagrilintide in a microsphere that is formed of a biodegradable polymer for a long-term elution. However, in this case, since a bioavailability of the cagrilintide encapsulated in the microsphere is low or a content of a drug included inside the microsphere is low, a large amount of the microsphere needs to be administered to exhibit the long-term effective pharmacological effect. However, when administering a large amount of the microsphere in vivo, there is a problem that it is difficult for a patient to administer by oneself (self-administer) due to the difficulty of subcutaneous injection and the possibility of pain and inflammatory response at an administration site is also very high.

### [Disclosure]

### [ Technical Problem]

The present invention is devised to solve the problems as described above and is directed to providing: a sustained-release microsphere including cagrilintide or a pharmaceutically acceptable salt thereof, a biodegradable polymer and an initial burst inhibiting agent that show a high bioavailability of the cagrilintide because of high content of the cagrilintide, and exhibit a long-term stable drug release; a sustained-release microsphere including cagrilintide or a pharmaceutically acceptable salt thereof, semaglutide or a pharmaceutically acceptable salt thereof, a biodegradable polymer, an initial burst inhibiting agent; or a combination of containing a sustained-release microsphere including the cagrilintide or the pharmaceutically acceptable salt thereof and the initial burst inhibiting agent, the biodegradable polymer and the initial burst inhibiting agent and a sustained-release microsphere including semaglutide or a pharmaceutically acceptable salt thereof, the biodegradable polymer, and the initial burst inhibiting agent; a pharmaceutical composition including the same; and a method of preparing the sustained-release microsphere.

### [Technical Solution]

Hereinafter, the present invention will be described in detail.

As used herein, the term "one or more types" refers to a "number" corresponding to one or more. In the present invention, when there is one or more types of configurations, it may preferably be one type, two or more types, three or more types, one to three types, or one to two types, but it is not limited thereto. The term "one or more types" may be used interchangeably with the term "at least one" in the present invention.

To achieve the above purpose,
as an aspect of the present invention, a pharmaceutical composition including cagrilintide sustained-release microspheres that include cagrilintide or a pharmaceutically acceptable salt thereof as an active ingredient, a biodegradable polymer, and an initial burst inhibiting agent is provided, wherein a content of the cagrilintide is 8 wt.% or more of the cagrilintide based on the total weight of the microsphere, and a content of the initial burst inhibiting agent is 10 ppm to 2000 ppm based on the total weight of the microsphere.

As an aspect of the present invention, a pharmaceutical composition including cagrilintide sustained-release microspheres that includes the cagrilintide or the pharmaceutically acceptable salt thereof and semaglutide or a pharmaceutically acceptable salt thereof as an active ingredient, the biodegradable polymer, and the initial burst inhibiting agent is provided, wherein the content of the cagrilintide is 8 wt.% or more of the cagrilintide based on the total weight of the microsphere, a content of the semaglutide is 8 wt.% or more of the semaglutide based on the total weight of the microsphere, and the content of the initial burst inhibiting agent is 10 ppm to 2000 ppm based on the total weight of the microsphere.

As an aspect of the present invention, a pharmaceutical composition including a combination of the cagrilintide sustained-release microsphere that includes the cagrilintide or the pharmaceutically acceptable salt thereof as an active ingredient, the biodegradable polymer, and the initial burst inhibiting agent and the semaglutide sustained-release microsphere that includes the semaglutide or the pharmaceutically acceptable salt thereof as the active ingredient, the biodegradable polymer, and the initial burst inhibiting agent is provided, wherein the content of the cagrilintide is 8 wt.% or more of the cagrilintide relative to the total weight of the microsphere, the content of the semaglutide is 8 wt.% or more of the semaglutide based on the total weight of the microsphere, and the content of the initial burst inhibiting agent is 10 ppm to 2000 ppm based on the total weight of the microsphere.

As another aspect, the biodegradable polymer may be at least one selected from a group consisting of: a polymer selected from a group consisting of polylactide (PLA), polyglycolide (PGA), polylactide-co-glycolide (PLGA), polydioxanone, polycaprolactone (PCL), polylactide-co-glycolide-co-caprolactone (PLGC), polylactide-co-hydroxymethyl glycolide (PLGMGA), polyalkylcarbonate, polytrimethylenecarbonate (PTMC), polylactide-co-trimethylenecarbonate (PLTMC), polyhydroxybutyric acid (PHB), polyhydroxybutyrate-co-hydroxyvalerate (PHBV), polyorthoester, polyanhydride, polyanhydride-co-imide, polypropylene fumarate, pseudo polyaminoacid, polyalkyl cyanoacrylate, polyphosphazene, polyphosphoester, polysaccharide, and block copolymers of poly(butylene succinate lactide) (PBSLA); a simple mixture of two or more types selected from the said polymers; a copolymer with said polymer and polyethylenglycol (PEG); and a polymer-sugar complex in which said polymer or said copolymer and sugar are bonded.

As still another aspect, the pharmaceutically acceptable salt of the cagrilintide may be sodium salt, acetate, benzoate, hydroxynaphthoate, napadisylate, or pamoate of the cagrilintide.

As still another aspect, an intrinsic viscosity of the biodegradable polymer may be 0.16 to 1.7 dL/g.

As still another aspect, an average particle size of the microsphere may be 5 µm to 100 µm.

As still another aspect, a span value of the microsphere may be 1.5 or less.

As still another aspect, a weight of the microsphere may be 20 to 1000 mg, 20 to 800 mg, 20 to 600 mg, 20 to 400 mg, 20 to 200 mg, 20 to 100 mg, 30 to 1000 mg, 30 to 800 mg, 30 to 600 mg, 30 to 400 mg, 30 to 200 mg, 30 to 100 mg, 40 to 1000 mg, 40 to 800 mg, 40 to 600 mg, 40 to 400 mg, 40 to 200 mg, 40 to 100 mg, 50 to 1000 mg, 50 to 800 mg, 50 mg to 600 mg, 50 mg to 400 mg, 50 mg to 200 mg, or 50 mg to 100 mg.

As still another aspect, the microsphere may further include one or more types of release control agents selected from a group consisting of butyric acid, valeric acid, caproic acid, enantic acid, caprylic acid, pelargonic acid, capric acid, undecylic acid, lauric acid, tridecylic acid, myristic acid, pentadecylic acid, palmitic acid, heptadecylic acid, stearic acid, nonadecylic acid, behenic acid, arachidic acid, isocrotonic acid, oleic acid, elaidic acid, sorbic acid, linoleic acid, arachidonic acid, benzoic acid, hydroxynaphthoic acid, napadisylic acid, naphthalenesulfonic acid, and pamoic acid.

As still another aspect, the microsphere may include 10 to 500 mg/kg of Na.

As still another aspect, the microsphere may include 1 to 100 mg/kg of P.

As still another aspect, the present invention provides a method of preparing a sustained-release microsphere that includes: (i) cagrilintide or a pharmaceutically acceptable salt thereof or (ii) cagrilintide or the pharmaceutically acceptable salt and semaglutide or a pharmaceutically acceptable salt thereof as an active ingredient; a biodegradable polymer; and an initial burst inhibiting agent.

As still another aspect, the method of preparing may use a continuous phase including the initial burst inhibiting agent.

As still another aspect, any substance that may be dissolved in the continuous phase during manufacturing of the microsphere to set a pH of the continuous phase to 7.0 or more may be used as the initial burst inhibiting agent.

As still another aspect, one or more types of substances selected from a group consisting of phosphate salt, hydroxide salt phosphide salt, phosphite salt, carbonate salt, bicarbonate salt, chromate salt, dichromate salt, oxide, oxalate salt, silicate salt, sulfate salt, sulfide salt, sulfite salt, tartrate salt, tetraborate salt, thiosulfate salt, arsenate salt, arsenite salt, citrate salt, ferricyanide salt, and nitride salt of an alkali metal, an alkaline-earth metal, or ammonium may be used as the initial burst inhibiting agent. As still another aspect, the initial burst inhibiting agent may be at least one selected from a group consisting of disodium phosphate, dipotassium phosphate, and diammonium phosphate.

As yet another aspect, a pharmaceutical composition including a biodegradable polymer and including a sustained-release microsphere, wherein a content of cagrilintide is 8 wt.% or more of the cagrilintide based on a total weight of the microsphere and an initial burst of a drug is 10% or less, and a method of preparing the same are provided.

### [Advantageous Effects]

According to a preparation example of the present invention, a sustained-release pharmaceutical composition including cagrilintide, a pharmaceutically acceptable salt thereof, and an initial burst inhibiting agent has an effect of minimizing pain of a patient and a side effect at an administration site that may occur during administration because not only a side effect caused by an initial burst is minimized as a rapid initial burst is inhibited despite including a high content of a drug relative to a particle size, but also a dosage is reduced while having a high bioavailability and exhibiting a long-acting drug efficacy for one month or more when administered in vivo.

### [Description of Drawings]

FIG. 1 is a scanning electron microscope photograph confirming morphological features of a microsphere manufactured in Examples 1-1 to 1-7.
FIG. 2 is a scanning electron microscope photograph confirming morphological features of a microsphere manufactured in Examples 2-1 to 2-4.
FIG. 3 is a scanning electron microscope photograph confirming morphological features of a microsphere manufactured in Examples 3-1 to 3-4.
FIG. 4 is a scanning electron microscope photograph confirming morphological features of a microsphere manufactured in Examples 4-1 to 4-3.
FIG. 5 is a scanning electron microscope photograph confirming morphological features of a co-encapsulation microsphere manufactured in Examples 5-1 and 5-6.
FIG. 6 is a scanning electron microscope photograph confirming morphological features of a microsphere manufactured in Comparative Examples 1 and 2.

### [Detailed Description]

Hereinafter, the present invention will be described in detail.

The present invention includes cagrilintide or a pharmaceutically acceptable salt thereof as an active ingredient.

Cagrilintide is a long-acting amylin analogue that targets a calcitonin receptor that is a receptor activator modifying proteins 1, 2, and 3 (RAMP 1-3). The cagrilintide is a non-selective agonist designed to be administered subcutaneously once a week at a low pH.

A structure of the cagrilintide is as shown in Chemical Formula 1 below.

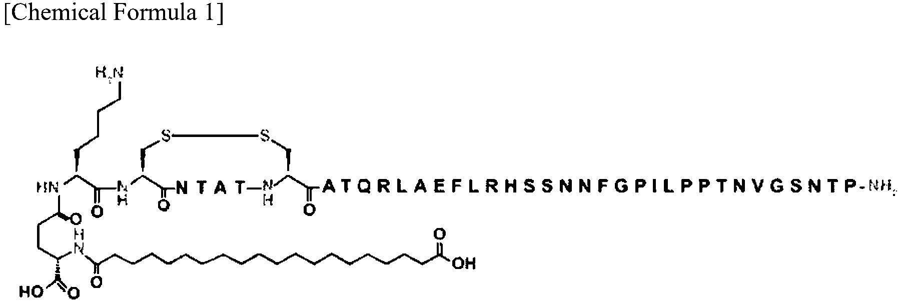

The cagrilintide may exist in a form of a salt, particularly in a form of a pharmaceutically acceptable salt. Any salt commonly used in the art may be used as the salt without limitation. As used herein, the term "pharmaceutically acceptable salt" refers to any organic or inorganic addition salt of the compound of which side effects caused by the salt do not reduce the beneficial effects of the active ingredient by a concentration that is relatively non-toxic and harmless to the patient. A specific example includes sodium salt, acetate, benzoate, hydroxynaphthoate, napadisylate, or pamoate of the cagrilintide, but it is not limited thereto.

The cagrilintide or the pharmaceutically acceptable salt thereof is the active ingredient of the present invention and may be in various forms, for example, in an amorphous form or a crystalline form.

The pharmaceutical composition according to the present invention may have an area under a blood concentration-time curve (AUC₀₋₂₄ₕᵣ) of the cagrilintide up to 24 hours after administration of 20% or less, 10% or less, 5% or less, 0.1 to 20%, 1 to 10%, or 1 to 5% relative to a total area under the blood concentration-time curve (AUCₜₒₜₐₗ).

The pharmaceutical composition according to the present invention may have an area under the blood concentration-time curve (AUC_{0-QXM}) of the cagrilintide up to an administration interval date after administration of 70% or less, 65% or less, 60% or less, 20 to 70%, 20 to 65%, 20 to 60%, 25 to 70%, 25 to 65%, 25 to 60%, 30 to 70%, 30 to 65% or 30 to 60% in a case of a human, and 95% or less, 93% or less, 90% or less, 50 to 95%, 50 to 93%, 50 to 90%, 55 to 95%, 55 to 93%, 55 to 90%, 60 to 95%, 60 to 93%, or 60 to 90% in a case of rats relative to the total area under the blood concentration-time curve (AUCₜₒₜₐₗ).

In addition, the pharmaceutical composition according to the present invention may be a pharmaceutical composition including a sustained-release microsphere in which an amount of the cagrilintide 12 wt.% or more of the cagrilintide based on the total weight of the microsphere and an initial burst of the drug is less than 10% within 24 hours.

A biodegradable polymer included in a cagrilintide sustained-release microsphere that is included in the pharmaceutical composition according to the present invention is selected from a group consisting of a polymer selected from a group consisting of polylactide (PLA), polyglycolide (PGA), polylactide-co-glycolide (PLGA), polydioxanone, polycaprolactone (PCL), polylactide-co-glycolide-co-caprolactone (PLGC), polylactide-co-hydroxymethyl glycolide (PLGMGA), polyalkylcarbonate, polytrimethylenecarbonate (PTMC), polylactide-co-trimethylenecarbonate (PLTMC), polyhydroxybutyric acid (PHB), polyhydroxybutyrate-co-hydroxyvalerate (PHBV), polyorthoester, polyanhydride, polyanhydride-co-imide, polypropylene fumarate, pseudo polyaminoacid, polyalkyl cyanoacrylate, polyphosphazene, polyphosphoester, polysaccharide, and poly(butylene succinate lactide) (PBSLA), a simple mixture of two or more types selected from said polymer, a copolymer with said selected polymer and polyethylenglycol (PEG), and a polymer-sugar complex in which the selected polymer or the copolymer and sugar are bonded.

In addition, that the biodegradable polymer is a simple mixture of two or more (i.e., a simple mixture including two or more of the selected polymers) may include two or more types of different types of polymers among the polymers exemplified non-limitingly, or may be a combination or blend of the same type of the polymers, but may be a combination of the polymers having different intrinsic viscosities and/or monomer ratios (e.g., a combination or blend of two or more poly (lactide-co-glycolide) having different intrinsic viscosities), or may be a same type of the polymers that differ in terminal groups (e.g., a same type of the polymers with an ester terminal group or an acid terminal group).

As a specific aspect, the pharmaceutical composition according to the present invention may include a microsphere including two or more types of the biodegradable polymers. As another specific embodiment, the pharmaceutical composition according to the present invention may include two or more types of microspheres each including at least one of two or more types of polymers selected from the biodegradable polymers.

In a case in which the biodegradable polymer is polylactide-co-glycolide, a molar ratio of lactide to glycolide in the copolymer may be 40:60 to 90:10, 45:55 to 85:15, or 50:50 to 75:25, for example, 45:55, 50:50, 75:25, or 85:15.

The biodegradable polymer may be a biodegradable polymer having an intrinsic viscosity of 0.16 to 1.7 dL/g, 0.2 to 1.3 dL/g, or 0.24 to 1.2 dL/g in consideration of factors such as release properties and a manufacturing process of a drug. The intrinsic viscosity refers to a viscosity measured at 0.1% (w/v) concentration in chloroform at 25 °C using an Ubbelohde viscometer.

When the intrinsic viscosity of the biodegradable polymer is less than 0.16 dL/g, a molecular weight of the polymer is insufficient, and thus it is difficult to exhibit a sustained-release effect of the cagrilintide or the pharmaceutically acceptable salt thereof, and when the intrinsic viscosity of the biodegradable polymer exceeds 1.7 dL/g, release of the cagrilintide or the pharmaceutically acceptable salt thereof may be excessively delayed. In addition, when manufacturing the microsphere using a polymer with high intrinsic viscosity, there is a problem that an excessive amount of a manufacturing solvent is used due to a high viscosity of the polymer, and thus it is difficult to manufacture a reproducible microsphere. Examples of a commercially available polymer having the above-described properties include RG502H, RG503H, RG504H, RG502, RG503, RG504, RG653H, RG752H, RG753H, RG752S, RG755S, RG750S, RG757S, RG858S, R202H, R203H, R205H, R202S, R203S, R205S, R206S, and R207S of Resomer^{®} series of Evonik, and PDL 02A, PDL 02, PDL 04, PDL 05, PDLG 7502A, PDLG 7502, PDLG 7507, PDLG 5002A, PDLG 5002, PDLG 5004A, and PDLG 5004 of Corbion.

An amount of the biodegradable polymer in the sustained-release microsphere including the cagrilintide or the pharmaceutically acceptable salt thereof and the initial burst inhibiting agent according to the present invention may select 60 wt.% or more, 62 wt.% or more, 65 wt.% or more, 67 wt.% or more, or 70 wt.% or more as an upper limit, and may select 92 wt.% or less, 91 wt.% or less, 90 wt.% or less, 89 wt.% or less, or 88 wt.% or less as a lower limit based on the total weight of the microsphere, or may be included in a range consisting of a combination of the upper and the lower limit. For example, the range may be 60 wt.% to 92 wt.%, 65 wt.% to 90 wt.%, or 70 wt.% to 88 wt.%, but it is not limited thereto.

An amount of the cagrilintide or the pharmaceutically acceptable salt thereof in the sustained-release microsphere including the cagrilintide or the pharmaceutically acceptable salt thereof and the initial burst inhibiting agent according to the present invention is preferably 8 wt.% or more, 12 wt.% or more, 14 wt.% or more, 37 wt.% or less, 35 wt.% or less, or 33 wt.% or less, 8 wt.% to 37 wt.%, 8 wt.% to 35 wt.%, 8 wt.% to 33 wt.%, 12 wt.% to 37 wt.%, 12 wt.% to 35 wt.%, 12 wt.% to 33 wt.%, 14 wt.% to 37 wt.%, 14 wt.% to 35 wt.%, or 14 wt.% to 33 wt.% as the cagrilintide based on the total weight of the microsphere. When the amount of cagrilintide or the pharmaceutically acceptable salt thereof in the microsphere is less than 8 wt.% based on the cagrilintide, an amount of the polymer used relative to the drug may be excessively high, and thus the bioavailability of the cagrilintide or the pharmaceutically acceptable salt thereof may decrease, and when the content is excessively high, the initial burst of the cagrilintide or the pharmaceutically acceptable salt thereof may increase, and thus it may not be preferable.

The pharmaceutical composition including the sustained-release microsphere according to the present invention may include the initial burst inhibiting agent in the microsphere in less than a certain amount.

The initial burst inhibiting agent in the present invention is a substance included in a continuous phase to inhibit a rapid release of the active ingredient in a process of manufacturing the microsphere, and it may exist in the microsphere according to the present invention in less than a certain amount. The continuous phase refers to a substance that enables a dispersed phase including an active ingredient such as the cagrilintide and the biodegradable polymer to be dispersed when an emulsion and solvent extraction evaporation method is used in the method of manufacturing the microsphere, and may be an aqueous solution including a surfactant in the present invention, but it is not limited thereto. The initial burst inhibiting agent according to the present invention is included in the continuous phase that is used in such a method of manufacturing, and accordingly, it may be included in the microsphere according to the present invention in a certain amount after manufacturing. Matters concerning the continuous phase may be applied in the same manner as those described in the method of manufacturing the microsphere according to the present invention described below.

As a specific aspect, the initial burst inhibiting agent may be included in 10 ppm or more, 20 ppm or more, 30 ppm or more, 40 ppm or more, 50 ppm or more, 100 ppm or more, 150 ppm or more, 200 ppm or more, 250 ppm or more, 200 ppm or more, 300 ppm or more, 350 ppm or more, 400 ppm or more, 450 ppm or more, 500 ppm or more, 550 ppm or more, 600 ppm or more, 650 ppm or more, 700 ppm or more, 750 ppm or more, 800 ppm or more, 850 ppm or more, 900 ppm or more, 950 ppm or more, 1000 ppm or more, 1100 ppm or more, 1200 ppm or more, 1300 ppm or more, 1400 ppm or more, or 1500 ppm or more as an upper limit, and 2000 ppm or less, 1900 ppm or less, 1800 ppm or less, 1700 ppm or less, 1600 ppm or less, 1500 ppm or less, 1400 ppm or less, 1300 ppm or less, 1200 ppm or less, 1100 ppm or less, 1000 ppm or less, 900 ppm or less, 800 ppm or less, 700 ppm or less, 600 ppm or less, or 500 ppm or less as a lower limit based on the total weight of the microsphere, and may be included in a range consisting of a combination of the upper limit and the lower limit. For example, the initial burst inhibiting agent may be included in 10 ppm to 2000 ppm, preferably 20 ppm to 1500 ppm, more preferably 20 ppm to 1000 ppm, and most preferably 30 ppm to 500 ppm based on the total weight of the microsphere.

As a specific aspect, any substance that enables the pH of the continuous phase to be set to 7 or more when dissolving in the continuous phase may be used as the initial burst inhibiting agent.

The initial burst inhibiting agent may be at least one selected from a group consisting of phosphate salt, hydroxide salt, phosphide salt, phosphite salt, carbonate salt, bicarbonate salt, chromate salt, dichromate salt, oxide, oxalate salt, silicate salt, sulfate salt, sulfide salt, sulfite salt, tartrate salt, tetraborate salt, thiosulfate salt, arsenate salt, arsenite salt, citrate salt, ferricyanide salt, and nitride salt of an alkali metal, an alkaline-earth metal, and ammonium, but it is not limited thereto.

As a specific aspect, the initial burst inhibiting agent may be at least one selected from a group consisting of phosphate salt of two or more alkali metals, carbonate salt of one or more alkali metals, and bicarbonate salt of one or more alkali metals, but it is not limited thereto.

As a more specific aspect, the phosphate salt of the two or more alkali metals refer to phosphate salt including two or more alkali metal ions, for example, disodium phosphate (Na₂HPO₄) or dipotassium phosphate (K₂HPO₄), bicarbonate salt of the one or more alkali metals refer to bicarbonate salt including one or more alkali metal ions, for example, sodium bicarbonate (NaHCO₃), the carbonate salt of the two or more alkali metals refer to carbonate salt including two or more alkali metal ions, for example, sodium carbonate (Na₂CO₃), and it is possible to use alone or by mixing two or more.

It is preferable for the microsphere including the cagrilintide or the pharmaceutically acceptable salt thereof and the initial burst inhibiting agent according to the present invention to have a uniform particle distribution of an average particle size of 5 µm to 100 µm, 5 µm to 90 µm, 5 µm to 80 µm, 10 µm to 90 µm, 10 µm to 80 µm, 15 µm to 100 µm, 15 µm to 90 µm, 15 µm to 80 µm, 70 µm to 100 µm, 70 µm to 90 µm, 70 µm to 80 µm, 60 µm to 100 µm, 60 µm to 80 µm, 60 µm to 70 µm, 20 µm to 90 µm, 20 µm to 70 µm, 20 µm to 60 µm, 30 µm to 80 µm, 30 µm to 60 µm, 40 µm to 70 µm, 40 µm to 50 µm, 30 µm to 40 µm, 20 µm to 30 µm, 5 µm to 30 µm, 5 µm to 20 µm, 10 µm to 20 µm, or 5 µm to 10 µm. The term "average particle size" used in the present invention refers to a particle size corresponding to 50% of the volume % in a particle size distribution curve, which refers to a median diameter and is represented as D50 or D(v, 0.5).

When the average particle size of the microsphere including the cagrilintide or the pharmaceutically acceptable salt thereof and the initial burst inhibiting agent is less than 5 µm, the release of the cagrilintide or the pharmaceutically acceptable salt drug thereof from the microsphere may be excessively fast, and thus it may not be preferable. When the average particle size exceeds 100 µm, an injection needle may become too thick when administering to a human body, thereby causing pain during injection or the drug may leak out from an injection site after injection, and thus it may not be preferable.

It is preferable for the microsphere including the cagrilintide or the pharmaceutically acceptable salt thereof and the initial burst inhibiting agent of the present invention to have a uniform particle distribution. The microsphere including the cagrilintide or the pharmaceutically acceptable salt thereof and the initial burst inhibiting agent having a uniform particle distribution has a smaller deviation during injection and may be administered in a more accurate amount compared to a non-uniform microsphere. It is preferable for a span value of the microsphere including the cagrilintide or the pharmaceutically acceptable salt thereof and the initial burst inhibiting agent of the present invention to be 1.5 or less. More preferably, it is preferable for the span value to be 1.2 or less. More specifically, the span value may be 1.5 or less, 1.2 or less, 0.1 to 1.5, 0.3 to 1.5, 0.5 to 1.5, 0.1 to 1.0, 0.4 to 1.0, 0.6 to 1.0, 0.2 to 0.8, or 0.4 to 0.8. The term "span value" used in the present invention is an index of the uniformity of the particle size of the microsphere and refers to a value obtained by a formula of Span value=(Dv0.9-Dv0.1)/Dv0.5. Here, Dv0.1 refers to a particle size corresponding to 10% of the volume% in the particle size distribution curve of the microsphere, Dv0.5 refers to a particle size corresponding to 50% of the volume% in the particle size distribution curve of the microsphere, and Dv0.9 refers to a particle size corresponding to 90% of the volume% in the particle size distribution curve of the microsphere.

The sustained-release microsphere including the cagrilintide or the pharmaceutically acceptable salt thereof and the initial burst inhibiting agent of the present invention is administered by an injection route, for example, subcutaneous injection and is especially self-administered, and thus it is preferable that the cagrilintide is released over a relatively long period of time. Preferably, the sustained-release microsphere in the pharmaceutical composition according to the present invention may release the cagrilintide or the pharmaceutically acceptable salt thereof for 1 month or more, 2 months or more, 3 months or more, 1 month to 2 months, 1 month to 3 months, 1 month to 4 months, 1 month to 5 months, 1 month to 6 months, 2 months to 6 months, 2 months to 5 months, 2 months to 4 months, 2 months to 3 months, 3 months to 5 months, or 3 months to 4 months, but it is not limited thereto. In addition, the sustained-release microsphere including the cagrilintide or the pharmaceutically acceptable salt thereof and the initial burst inhibiting agent of the present invention not particularly limited in the release pattern, but it is preferable that the cagrilintide or the pharmaceutically acceptable salt thereof is released for 24 hours when administered in vivo at a release rate of less than 10%, less than 15%, or less than 20% as an upper limit, or at a release rate of 0.001 % or more, 0.01% or more, 0.1% or more, or 1% or more as a lower limit, or at a release rate in a range of a combination of the upper limit and the lower limit.

In addition, a total amount (total weight) of the sustained-release microsphere formulation including the cagrilintide or the pharmaceutically acceptable salt thereof and the initial burst inhibiting agent in the pharmaceutical composition of the present invention may be 20 to 1000 mg, 20 to 800 mg, 20 to 600 mg, 20 to 400 mg, 20 to 200 mg, 20 to 100 mg, 30 to 1000 mg, 30 to 800 mg, 30 to 600 mg, 30 to 400 mg, 30 to 200 mg, 30 to 100 mg, 40 to 1000 mg, 40 to 800 mg, 40 to 600 mg, 40 to 400 mg, 40 mg to 200 mg, 40 mg to 100 mg, 50 mg to 1000 mg, 50 mg to 800 mg, 50 mg to 600 mg, 50 mg to 400 mg, 50 mg to 200 mg, or 50 mg to 100 mg. As the sustained-release microsphere including the cagrilintide or the pharmaceutically acceptable salt thereof and the initial burst inhibiting agent are included in the composition in the above range, the composition according to the present invention has an advantage of minimizing an inflammatory response at a site of administration and enabling self-administration by the patient.

The microsphere including the cagrilintide or the pharmaceutically acceptable salt thereof and the initial burst inhibiting agent included in the composition of the present invention may further include a release control agent. An example of a substance used as the release control agent may be at least one selected from butyric acid, valeric acid, caproic acid, enantic acid, caprylic acid, pelargonic acid, capric acid, undecylic acid, lauric acid, tridecylic acid, myristic acid, pentadecylic acid, palmitic acid, heptadecylic acid, stearic acid, nonadecylic acid, behenic acid, arachidic acid, isocrotonic acid, oleic acid, elaidic acid, sorbic acid, linoleic acid, arachidonic acid, benzoic acid, hydroxynaphthoic acid, napadisylic acid, naphthalenesulfonic acid, and pamoic acid, but it is not limited thereto. Preferably, the release control agent may be hydroxynaphthoic acid, napadisylic acid, or pamoic acid, but it is not limited thereto.

The pharmaceutical composition including the microsphere that includes the cagrilintide or the pharmaceutically acceptable salt thereof and the initial burst inhibiting agent according to the present invention may be formulated into various forms of formulations and for example, it may be a dosage form of a known parenteral administration formulation. Accordingly, the pharmaceutical composition according to the present invention may further include a thickening agent, a stabilizing agent, an isotonic agent, a surfactant, an excipient and/or a carrier in addition to the cagrilintide or the pharmaceutically acceptable salt thereof and the initial burst inhibiting agent. An available isotonic agent that may be used may be a water-soluble excipient or sugar such as mannitol, sucrose, sorbitol, trehalose, lactose, sodium chloride, etc., and examples of the thickening agent may include carmellose sodium, carboxymethyl cellulose sodium, povidone, etc. In addition, sodium dihydrogen phosphate, anhydrous citric acid, sodium hydroxide, sodium chloride, etc., may be used as the stabilizing agent.

The pharmaceutical composition according to the present invention may be administered in a therapeutically effective amount of the cagrilintide, for example, an effective amount for treating diabetes, specifically type 2 diabetes, beta-cell dysfunction, hypertension, hyperlipidemia, obesity, non-alcoholic steatohepatitis, or degenerative neurological diseases such as Alzheimer's disease and Parkinson's disease. The therapeutically effective amount of the cagrilintide may be assessed by a physician. The pharmaceutical composition according to the present invention including the cagrilintide may be administered once a month or once a quarter. In some specific examples, a monthly dosage of the composition according to the present invention may be 1 mg to 100 mg, 1 mg to 80 mg, 1 mg to 60 mg, 1 mg to 30 mg, 1 mg to 20 mg, 1 mg to 10 mg, 1 mg to 8 mg, 1 mg to 6 mg, 2 mg to 100 mg, 2 mg to 80 mg, 2 mg to 60 mg, 2 mg to 30 mg, 2 mg to 20 mg, 2 mg to 10 mg, 2 mg to 8 mg, 2 mg to 6 mg, 4 mg to 100 mg, 4 mg to 80 mg, 4 mg to 60 mg, 4 mg to 30 mg, 4 mg to 20 mg, 8 mg to 100 mg, 8 mg to 80 mg, 8 mg to 60 mg, 8 mg to 30 mg, 10 mg to 100 mg, 10 mg to 80 mg, 10 mg to 60 mg, 10 mg to 30 mg, 20 mg to 100 mg, 20 mg to 80 mg, 20 mg to 60 mg, or 20 mg to 30 mg based on the cagrilintide. The pharmaceutical composition according to the present invention including the microsphere that includes the cagrilintide or the pharmaceutically acceptable salt thereof and the initial burst inhibiting agent according to the present invention may also be administered parenterally, for example, by subcutaneous injection. The pharmaceutical composition according to the present invention may be composed of a drug part including the microsphere that includes the cagrilintide or the pharmaceutically acceptable salt thereof and the initial burst inhibiting agent and a solvent part used to suspend the microsphere, and may be in a form of a dual chamber syringe having the drug part in one chamber and the solvent part in the other chamber, or in a form of a prefilled syringe in which the drug part is suspended in the solvent part. When composed in the prefilled syringe form in which the drug part is suspended in the solvent part in a prefilled syringe, the solvent part used may be an injectable oil including a medium-chain oil, mineral oil, etc.

In a specific embodiment, the sustained-release microsphere including the cagrilintide or the pharmaceutically acceptable salt thereof and the initial burst inhibiting agent included in the pharmaceutical composition according to the present invention has a high drug relative to the content of the microsphere, and at the same time, inhibits an excessive initial burst of the drug that may cause fatal side effects and exhibits sufficient drug efficacy as an amylin analogue for a desired period of time due to the high bioavailability, and thus it is useful for the prevention or treatment of diabetes, specifically type 2 diabetes, beta-cell dysfunction, hypertension, hyperlipidemia, obesity, non-alcoholic steatohepatitis, or Alzheimer's disease.

As still another aspect, the present invention provides a pharmaceutical composition including the sustained-release microsphere that includes the cagrilintide or the pharmaceutically acceptable salt thereof and the initial burst inhibiting agent and a sustained-release microsphere that includes semaglutide or a pharmaceutically acceptable salt thereof and the biodegradable polymer for the prevention or treatment of diabetes, type 2 diabetes, beta-cell dysfunction, obesity, non-alcoholic steatohepatitis, Alzheimer's disease or neurodegenerative diseases,.

The aforementioned matters may be applied to the sustained-release microsphere including the cagrilintide or the pharmaceutically acceptable salt thereof and the initial burst inhibiting agent as it is.

In a specific embodiment, a pharmaceutical composition including a semaglutide sustained-release microsphere which is a microsphere including the semaglutide included in the pharmaceutical composition according to the present invention is provided, wherein a content of the semaglutide is 8 wt.% or more of the semaglutide based on the total weight of the microsphere.

It is preferable that a content of the semaglutide or the pharmaceutically acceptable salt thereof in the sustained-release microsphere including the semaglutide or the pharmaceutically acceptable salt thereof according to the present invention is 8 wt.% or more, 12 wt.% or more, 14 wt.% or more, 37 wt.% or less, 35 wt.% or less, or 33 wt.% or less, 8 wt.% to 37 wt.%, 8 wt.% to 35 wt.%, 8 wt.% to 33 wt.%, 12 wt.% to 37 wt.%, 12 wt.% to 35 wt.%, 12 wt.% to 33 wt.%, 14 wt.% to 37 wt.%, 14 wt.% to 35 wt.%, or 14 wt.% to 33 wt.% of the semaglutide based on the total weight of the microsphere.

As still another aspect, the present invention provides a pharmaceutical composition including the sustained-release microsphere that includes the cagrilintide or the pharmaceutically acceptable salt thereof and the semaglutide or the pharmaceutically acceptable salt thereof, and the initial burst inhibiting agent at the same time for the prevention or treatment of diabetes, type 2 diabetes, beta-cell dysfunction, obesity, non-alcoholic steatohepatitis, Alzheimer's disease or neurodegenerative diseases.

In a specific embodiment, the pharmaceutical composition including the sustained-release microsphere that includes the cagrilintide or the semaglutide which is the sustained-release microsphere including the cagrilintide or the pharmaceutically acceptable salt thereof, the semaglutide or the pharmaceutically acceptable salt thereof, and the initial burst inhibiting agent included in the pharmaceutical composition according to the present invention is provided, wherein an amount of the cagrilintide and the semaglutide is 4 wt.%, respectively, based on the total weight of the microsphere and a content of the initial burst inhibiting agent is 10 ppm to 2000 ppm based on the total weight of the microsphere.

It is preferable that a content of each of the cagrilintide or the pharmaceutically acceptable salt thereof and the semaglutide or the pharmaceutically acceptable salt thereof in the sustained-release microsphere including the cagrilintide or the pharmaceutically acceptable salt thereof, the semaglutide or the pharmaceutically acceptable salt thereof, and the initial burst inhibiting agent according to the present invention is 4 wt.% or more, 6 wt.% or more, 7 wt.% or more, 20 wt.% or less, 18.5 wt.% or less, 17.5 wt.% or less, or 16.5 wt.% or less, 4 wt.% to 20 wt.%, 4 wt.% to 18.5 wt.%, 4 wt.% to 17.5 wt.%, or 4 wt.% to 16.5 wt.%, 6 wt.% to 20 wt.%, 6 wt.% to 18.5 wt.%, 6 wt.% to 17.5 wt.%, or 6 wt.% to 16.5 wt.%, 7 wt.% to 20 wt.%, 7 wt.% to 18.5 wt.%, 7 wt.% to 17.5 wt.%, or 7 wt.% to 16.5 wt.%, respectively, as the cagrilintide and the semaglutide based on the total weight of the microsphere.

In addition, an amount of the initial burst inhibiting agent in the sustained-release microsphere including the cagrilintide or the pharmaceutically acceptable salt, the semaglutide or the pharmaceutically acceptable salt thereof, and the initial burst inhibiting agent according to the present invention is the same as that for the above-described cagrilintide microsphere.

In addition, all matters regarding the biodegradable polymer and an amount thereof, etc., regarding the microsphere may be applied to the matters regarding the cagrilintide microsphere as it is.

Maximum efficacy that the semaglutide, which is a GLP-1 receptor agonist, may be limited by tolerability. When the dosage is increased, adverse reactions such as nausea and vomiting become more prominent. An amylin receptor agonist therapy is limited by tolerability in almost the same way as a GLP-1 receptor agonist therapy (and by similar adverse reactions such as nausea and vomiting), and there is an expectation that it may prolong an action of the amylin, and thus a combination of the cagrilintide that is the amylin receptor agonist and the semaglutide that is the GLP-1 receptor agonist is needed.

However, it is known that the semaglutide and the cagrilintide may not be formulated into a same pharmaceutical dosage form. In order to ensure physical stability as well as chemical stability, the semaglutide needs to be formulated at a pH of 7 to 8, for example, at approximately pH 7.4. In order to ensure physical stability as well as chemical stability, the cagrilintide needs to be formulated at a pH of 3.5 to 4.5, for example, at approximately pH 4.0. Due to the significant difference in formulation, the two pharmaceutical dosage forms including the semaglutide and the cagrilintide, respectively, needs to be stored separately until administration. Therefore, in order to co-administer them, they need to be administered separately or mixed when using, and since they are administered through subcutaneous injection, they may cause more pain and discomfort for a patient, especially when administered separately. Therefore, when they may be administered as a single injection rather than as two injections, compliance with treatment will be improved, ultimately improving a desired pharmacological outcome. In addition, administering the cagrilintide and the semaglutide as a single injection rather than administering them separately may affect efficacy of these active pharmaceutical substances.

In relation to this, there have been attempts to develop a dual chamber device including these drugs, but there was a problem that the drugs are not stabilized and particles are generated during storage in the dual chamber device.

However, in a case of simultaneously encapsulating the cagrilintide and the semaglutide in the microsphere or combining the microsphere in which each ingredient is encapsulated as in the present invention, while the stability of the drug is secured, there is no need to adjust the above-mentioned separate pH and select buffer solutions, and thus a cost problem caused by manufacturing with separate formulations and the inconvenience of mixing immediately before administration may be eliminated.

As still another aspect, the present invention provides a method of preparing the sustained-release microsphere including the cagrilintide or the pharmaceutically acceptable salt thereof as the active ingredient, the biodegradable polymer, and the initial burst inhibiting agent.

In another aspect, the present invention provides a method of preparing a sustained-release microspheres having a significantly inhibited initial burst despite including a high content of the cagrilintide or the pharmaceutically acceptable salt thereof.

Hereinafter, a method of preparing a sustained-release microsphere injection of the present invention will be specifically described.

The sustained-release microsphere according to the present invention may be prepared, for example, by using "emulsion solvent extraction and evaporation method", but the method of preparing is not limited thereto.

As an aspect of a specific method of preparing, the present invention provides a method of preparing the sustained-release microsphere with an oil-in-water (O/W) emulsion including the following steps:
(a) preparing an oil phase (O phase) as a dispersed phase obtained by dissolving (i) cagrilintide or a pharmaceutically acceptable salt thereof, or (ii) cagrilintide or a pharmaceutically acceptable salt thereof and semaglutide or a pharmaceutically acceptable salt thereof as an active ingredient, and one or more types of biodegradable polymers in an organic solvent;
(b) preparing a continuous phase (W phase) by adding an initial burst inhibiting agent to an aqueous solution including a surfactant, and adding the dispersed phase of the step (a) to prepare a dispersed phase in an emulsion state;
(c) extracting and evaporating an organic solvent from the dispersed phase in the emulsion state manufactured in the step (b) in the continuous phase (W phase) to form a microsphere;
(d) recovering the microsphere.

In the step (a), when the cagrilintide or the pharmaceutically acceptable salt thereof and the semaglutide or the pharmaceutically acceptable salt are used as the active ingredient, a co-encapsulated microsphere including the cagrilintide and the semaglutide at the same time may be prepared.

As another aspect, the present invention provides a method of preparing the sustained-release microsphere with an water-in-oil-in-water (W/O/W) emulsion including the following steps:
(a') preparing a an aqueous phase (W1 phase) by dissolving (i) cagrilintide or a pharmaceutically acceptable salt thereof or (ii) cagrilintide or a pharmaceutically acceptable salt thereof and semaglutide or a pharmaceutically acceptable salt thereof as an active ingredient in an aqueous solution and preparing an oil phase (O phase) by dissolving one or more types of biodegradable polymers in an organic solvent to prepare a primary W1/O emulsion as a dispersed phase in which the aqueous phase (W1 phase) and the oil phase (O phase) are mixed;
(b') preparing a continuous phase (W2 phase) by adding an initial burst inhibiting agent to an aqueous solution including a surfactant and adding the dispersed phase of the step (a') to manufacture a dispersed phase in a secondary W1/O/W2 emulsion state;
(c') extracting and/or evaporating an organic solvent from the dispersed phase in the secondary W1/O/W2 emulsion state of the step (b') in the continuous phase (W2 phase) to form a microsphere;
(d') recovering the microsphere.

In the step (a'), when the cagrilintide or the pharmaceutically acceptable salt thereof and the semaglutide or the pharmaceutically acceptable salt are used as the active ingredient, a co-encapsulated microsphere including the cagrilintide and the semaglutide at the same time may be prepared.

When two types of the drugs are included when preparing the aqueous phase in the step (a'), the two types of drugs may be dissolved simultaneously in one type of the aqueous solution to prepare the aqueous phase (W phase), or the two types of drugs may be dissolved in each aqueous solution to prepare an aqueous phase a (W1a phase) and an aqueous phase b (W1b phase). When the aqueous phase a (W1a phase) and the aqueous phase b (W1b phase) are prepared, they may be mixed simultaneously or mixed sequentially with the oil phase (O phase) to prepare the W1/O emulsion as the dispersed phase.

As still another aspect, a pH of the continuous phase used in the methods of manufacturing may be 7 or more.

In the manufacturing of the sustained-release microsphere including the cagrilintide or the pharmaceutically acceptable salt thereof and the initial burst inhibiting agent according to the present invention, the sustained-release microsphere includes a high content of the cagrilintide or the pharmaceutically acceptable salt thereof relative to the weight of the microsphere, and at the same time, inhibits excessive initial burst of the cagrilintide or the pharmaceutically acceptable salt thereof, has high bioavailability, and may use the following biodegradable polymers to release at a constant concentration for a desired long period of time, for example, 1 month or more, 3 months or more, 1 month to 2 months, 1 month to 3 months, 1 month to 4 months, 1 month to 5 months, 1 month to 6 months, 2 months to 6 months, 2 months to 5 months, 2 months to 4 months, 2 months to 3 months, 3 months to 5 months, or 3 months to 4 months, but it is not limited thereto. Specifically, it is preferable to use one or more types of polymers, preferably two or more types of polymers selected from a group consisting of a polymer selected from a group consisting of polylactide (PLA), polyglycolide (PGA), polylactide-co-glycolide (PLGA), polydioxanone, polycaprolactone (PCL), polylactide-co-glycolide-co-caprolactone (PLGC), polylactide-co-hydroxymethyl glycolide (PLGMGA), polyalkylcarbonate, polytrimethylenecarbonate (PTMC), polylactide-co-trimethylenecarbonate (PLTMC), polyhydroxybutyric acid (PHB), polyhydroxybutyrate-co-hydroxyvalerate (PHBV), polyorthoester, polyanhydride, polyanhydride-co-imide, polypropylene fumarate, pseudo polyaminoacid, polyalkyl cyanoacrylate, polyphosphazene, polyphosphoester, polysaccharide, and poly(butylene succinate lactide) (PBSLA), a simple mixture of two or more types selected from said polymer, a copolymer with said selected polymer and polyethylenglycol (PEG), and a polymer-sugar complex in which said selected polymer or the copolymer and sugar are bonded. As a specific embodiment, in the method of preparing according to the present invention, poly (lactide-co-glycolide) and/or a polylactide polymer may be used as the biodegradable polymer.

In the present invention, two or more types of different biodegradable polymers may include two or more polymers having different repeating units constituting the polymers and two or more polymers having different molar ratios of the repeating units when including two or more repeating units. As an example, the microsphere may be a mixture of a microsphere including poly-lactide-co-glycolide and a microsphere including a polylactide polymer, or may be a microsphere including both the poly-lactide-co-glycolide and the polylactide polymer.

In addition, as a specific example, when there are two types of the biodegradable polymers that are different from each other, a content ratio of the biodegradable polymers may be a weight ratio of 0.5:10 to 10:0.5, 0.5:8 to 8:0.5, 1:10 to 10:1, 1:4 to 4:1, 1:3 to 3:1, or 1:2 to 2:1, but it is not limited thereto.

In addition, unless otherwise defined, the description of the biodegradable polymer described in the microsphere according to the present invention may be applied as it is.

As a more specific aspect, when the polylactide-co-glycolide is used as the two or more types of biodegradable polymers to manufacture the sustained-release microsphere including the cagrilintide or the pharmaceutically acceptable salt thereof and the initial burst inhibiting agent according to the present invention, at least one biodegradable polymer having an intrinsic viscosity of 0.16 dL/g to 0.45 dL/g may be included.

In a case of the polylactide-co-glycolide, a molar ratio of lactide to glycolide in the copolymer may be 40:60 to 90:10, 45:55 to 85:15, or 50:50 to 75:25, for example, 45:55, 50:50, 75:25, or 85:15.

The organic solvent used to dissolve at least one biodegradable polymer in the step (a) or (a') of the specific method of manufacturing is one or more organic solvents. In addition, a mixed organic solvent in which two or more types of organic solvents are mixed may be used as the organic solvent. As a specific aspect, the mixed solvent may be a mixed solvent of an organic solvent that is miscible with water and an organic solvent that is not miscible with water. In this case, it is preferable to use an organic solvent with a water-immiscible property in 50% (v/v) or more, 60% (v/v) or more, 50 to 99.9% (v/v), 50 to 90% (v/v), 50 to 80% (v/v), 50 to 70% (v/v), 60 to 90% (v/v), or 60 to 80% (v/v). By using the water-immiscible property of the organic solvent, the dispersed phase may be homogeneously mixed in the continuous phase including the surfactant in the step (b) or (b') described later to form the emulsion. A type of the organic solvent that dissolves one or more types of the biodegradable polymers is not particularly limited, but preferably a mixed solvent of one or more solvents selected from a group consisting of dichloromethane, chloroform, ethyl acetate, methyl ethyl ketone, acetone, acetonitrile, dimethyl sulfoxide, dimethyl formamide, n-methyl pyrrolidone, acetic acid, methyl alcohol, ethyl alcohol, propyl alcohol, and benzyl alcohol, more preferably one type of solvent selected from dichloromethane and ethyl acetate, and one or more types of organic solvents selected from dimethyl sulfoxide, n-methyl pyrrolidone, methyl alcohol, and acetic acid may be used. In an embodiment, dichloromethane and acetic acid (glacial acetic acid) may be used by mixing as the organic solvent.

The method for homogeneously mixing the continuous phase including the dispersed phase and the surfactant in the step (b) or (b') is not particularly limited, but it may be performed using a high-speed stirrer, an inline mixer, a membrane emulsion method, a microfluidics emulsion method, an ultrasonic mixer, or a static mixer, alone or by using two or more types. When forming an emulsion using the high-speed stirrer, the inline mixer, the ultrasonic mixer, or the static mixer, it is difficult to obtain a uniform emulsion, and thus it is preferable to additionally perform a sieving process, etc. between steps (c) and (d) or between steps (c') and (d') described later.

The type of the surfactant used in the step (b) or (b') is not particularly limited, and any type that may help form a dispersed phase with a stable droplet inside the continuous phase may be used. As the surfactant, a surfactant selected from a group consisting of polyvinyl alcohol, methyl cellulose, polyvinylpyrrolidone, carboxymethylcellulose, lecithin, gelatin, polyoxyethylene sorbitan fatty acid ester, and polyoxyethylene castor oil derivative, etc., may be used alone or by mixing two or more types.

In the step (b) or (b'), a content of the surfactant in the continuous phase including the surfactant may be 0.01 w/v% to 20 w/v%, preferably 0.03 w/v% to 18 w/v%, 0.05 w/v% to 15 w/v%, 0.07 w/v% to 10 w/v%, or 0.1 w/v% to 5 w/v%, based on a total volume of the continuous phase including the surfactant. When the content of the surfactant is less than 0.01 w/v%, a droplet-shaped dispersed phase or the emulsion may not be formed inside the continuous phase, and when the content of the surfactant exceeds 20 w/v%, it may be difficult to remove the surfactant after fine particles are formed inside the continuous phase due to the excessive amount of the surfactant.

An aqueous solution including the surfactant and the initial burst inhibiting agent may be used as the continuous phase used in the step (b) or (b'). In addition, the continuous phase may also be used by adding to the aqueous solution including the surfactant so as to reach a final concentration of the initial burst inhibiting agent. As the initial burst inhibiting agent is used, the initial burst of the microsphere enclosed with a high content of a drug may be controlled, and a substance forming a multivalent anion may be used as the initial burst inhibiting agent.

The type of the initial burst inhibiting agent is not particularly limited, and any basic salt that may be dissolved in the continuous phase and maintains a pH of 7.0 or more may be used. The initial burst inhibiting agent may use one or more types of substances selected from phosphate salt, hydroxide salt, phosphide salt, phosphite salt, carbonate salt, bicarbonate salt, chromate salt, dichromate salt, oxide, oxalate salt, silicate salt, sulfate salt, sulfide salt, sulfite salt, tartrate salt, tetraborate salt, thiosulfate salt, arsenate salt, arsenite salt, citrate salt, ferricyanide salt, and nitride salt of an alkali metal, an alkaline-earth metal, or ammonium.

As a specific aspect, the initial burst inhibiting agent may be at least one selected from a group consisting of phosphate salt of two or more alkali metals, bicarbonate salt of one or more alkali metals, and carbonate salt of one or more alkali metals, but it is not limited thereto.

As a more specific aspect, the phosphate salt of the two or more alkali metals is disodium phosphate (Na₂HPO₄) or dipotassium phosphate (K₂HPO₄), the bicarbonate salt of the one or more alkali metals is sodium bicarbonate (NaHCO₃), and the carbonate salt of the one or more alkali metals is sodium carbonate (Na₂CO₃), and it is possible to use alone or by mixing two or more types among them.

In the continuous phase, the final concentration of the initial burst inhibiting agent may be 0.1 to 5.0 (w/v)%, preferably 0.1 to 4.0 (w/v)%, more preferably 0.2 to 4.0 (w/v)%, still more preferably 0.3 to 3.5 (w/v)%, particularly preferably 0.4 to 3.0 (w/v)%, and particularly more preferably 0.5 to 2.5 (w/v)%.

When a content of the initial burst inhibiting agent exceeds 5.0 w/v%, a phenomenon of an emulsion droplet bursting may occur during the manufacturing of the microsphere, and conversely, when the content is lower than 0.1 w/v%, the initial burst of the microsphere including the high content of the cagrilintide may not be sufficiently inhibited.

When the initial burst inhibiting agent is included in the continuous phase during the manufacturing of the microsphere, the initial burst in the microsphere including 12 (w/w)% or more of the cagrilintide may be reduced preferably to 10% or less, more preferably to 8% or less, and most preferably to 5% or less.

The continuous phase used in the step (b) or (b') may further include at least one selected from a group consisting of methyl alcohol, ethyl alcohol, propyl alcohol, and ethyl acetate control an extraction rate of the organic solvent from the dispersed phase in an emulsion state.

The initial burst inhibiting agent may be added at a time point of preparing the continuous phase in the step (b) or (b'), and the initial burst inhibiting agent may also be added at any time point during supplying of the continuous phase in the step (c) or (c'), and an adding method is not limited.

In addition, the pH of the continuous phase may be 7.0 or more, 7.2 or more, 7.4 or more, 8.0 or more, 8.5 or more, 9.0 or more, 7.0 to 9.0, or 7.0 to 8.5, but it is not limited thereto. When the pH of the continuous phase is controlled to be within the range, the bioavailability of the microsphere including the cagrilintide or the pharmaceutically acceptable salt thereof may be further increased.

In the step (c) or (c'), when the emulsion including the continuous phase that includes the droplet-shaped dispersed phase and the surfactant is maintained or stirred at a temperature below the boiling point of the organic solvent for a certain period of time, for example, 2 hours to 48 hours, the organic solvent may be extracted to the continuous phase from the cagrilintide which is the droplet-shaped dispersed phase or the pharmaceutically acceptable salt thereof and a polymer solution. A part of the organic solvent extracted to the continuous phase may evaporate from a surface thereof. While the organic solvent is extracted and evaporated from the droplet-shaped cagrilintide or the pharmaceutically acceptable salt thereof and the polymer solution, the droplet-shaped dispersed phase may be solidified to form the microsphere.

In order to additionally and efficiently remove the organic solvent in the step (c) or (c'), temperature of the continuous phase may be heated for a certain period of time. The heating temperature is not limited and may be appropriately controlled by those having an ordinary skill in the art depending on the organic solvent that is used. For example, when using dichloromethane as the organic solvent, heat may be applied so as to maintain at 30 °C or higher, 40 °C or higher, 45 °C or higher, 30 to 50 °C, 40 to 50 °C, or 45 °C.

In the step (d) or (d'), a method of recovering the microsphere including the cagrilintide or the pharmaceutically acceptable salt thereof and the initial burst inhibiting agent may be performed using various known techniques, for example, methods such as filtration or centrifugation may be used.

Between the steps (c) and (d) or between the steps (c') and (d'), the residual surfactant may be removed through filtration and washing, and filtered again to recover the microsphere.

The washing step for removing the residual surfactant may be normally performed using water, and the washing step may be repeated for several times.

In addition, as described above, the sieving process may be additionally used between the steps (c) and (d) or between the steps (c') and (d') to obtain a uniform microsphere. The sieving process may be performed using a known technique, and microspheres with small and large particles may be filtered out using sieves of different sizes to obtain the microsphere with a uniform size.

The method of manufacturing of the present invention may dry the obtained microsphere using a conventional drying method after the step (d) or step (d') or after the filtering and washing step to finally obtain a dried microsphere.

In addition to the above-described matters, all matters including the cagrilintide, the initial burst inhibiting agent, the biodegradable polymer and contents thereof that are not separately defined may be applied as the matters defined in the pharmaceutical composition.

According to the method of preparing of the present invention, a sustained-release microsphere injection the cagrilintide or the pharmaceutically acceptable salt thereof in which the cagrilintide or the pharmaceutically acceptable salt drug thereof is maintained at an effective concentration for a desired period of time without a initial burst of the drug and has a high bioavailability may be manufactured. In addition, the sustained-release microsphere injection with uniform particles having good administration ability including the cagrilintide or the pharmaceutically acceptable salt thereof and the initial burst inhibiting agent may be prepared.

As still another aspect, the present invention provides a method of preparing the sustained-release microsphere including the cagrilintide or the pharmaceutically acceptable salt, and the semaglutide or the pharmaceutically acceptable salt thereof, and the initial burst inhibiting agent.

As yet another aspect, the present invention provides a method of preparing the sustained-release microsphere having a significantly inhibited initial burst despite including a high content cagrilintide or the pharmaceutically acceptable salt thereof, and the semaglutide or the pharmaceutically acceptable salt thereof.

A specific description of the method of manufacturing the sustained-release microsphere injection including the cagrilintide or the pharmaceutically acceptable salt thereof, the semaglutide or the pharmaceutically acceptable salt thereof, and the initial burst inhibiting agent is the same as the method of preparing the sustained-release microsphere including the cagrilintide or the pharmaceutically acceptable salt thereof and the initial burst inhibiting agent described above except for adding the semaglutide or the pharmaceutically acceptable salt thereof as the active ingredient.

The sustained-release microsphere injection including the cagrilintide or the pharmaceutically acceptable salt thereof, the semaglutide or the pharmaceutically acceptable salt thereof, and the initial burst inhibiting agent thereof may be manufactured, for example, by using "emulsion solvent extraction and evaporation method", but the method of manufacturing is not limited thereto.

The sustained-release microspheres according to the present invention may be blended with two or more types of drug microspheres containing the same drug but differing in one or more of the composition and manufacturing conditions, or may be blended with two or more types of drug microspheres containing different drugs. In the case of microsphere blending comprising two or more types of drug microspheres, it may be performed for the purpose of optimizing the release profile of the drug, controlling the release period, and the like.

Specifically, the different composition and manufacturing conditions may be at least one selected from a group consisting of usage amount a drug, polymer type, particle size distribution (e.g., average particle size), circularity, usage amount of a polymer, type and usage amount of a dispersed phase solvent, type and usage amount of co-solvent, type and usage amount of a continuous phase, temperature and time of solidification, theoretical content of a drug, etc., but are not limited thereto.

The microsphere blending may be, for example, mixing the drug microspheres having one or more different composition and manufacturing conditions at a specific ratio. Specifically, when blending the microsphere including the cagrilintide or the pharmaceutically acceptable salt thereof and the microsphere including the semaglutide or the pharmaceutically acceptable salt thereof, a mixing ratio (weight ratio or ratio of number of microspheres) may be appropriately adjusted in consideration of the bioavailability of the active ingredient of each microsphere (i.e., cagrilintide or semaglutide). For example, the microspheres may be mixed at a weight ratio or number ratio of microspheres comprising cagrilintide or a pharmaceutically acceptable salt thereof to microspheres comprising semaglutide or a pharmaceutically acceptable salt thereof of 1:100, 1:70, 1:50, 1:30, 1:10, 1:5, 1:2, 1:1, 2:1, 5:1, 10:1, 30:1, 50:1, 70:1, or 100:1.

As a specific example, the two or more types of different drug microspheres may be drug microspheres that differ in components and composition ratios (hereinafter, drug microspheres with different compositions) and/or drug microspheres that differ in drug release properties, and for example, at least one selected from a group consisting of the type of a polymer of a microsphere, the content of a polymer, the content of a drug, etc. may be different. The difference in the type of a polymer of the microsphere may be a difference in at least one selected from a group consisting of a repeating unit of a polymer, a terminal group of a polymer, a molecular weight of a polymer, and an intrinsic viscosity of a polymer, etc.

The microsphere blending may be performed by manufacturing each microsphere separately and mixing them, or by manufacturing the microspheres in a single manufacturing process. Specifically, an O/W emulsion method of manufacturing a microsphere blending in a single manufacturing process may be a manufacturing method including the following steps 1 to 4 as an example.

Separately preparing two or more types of dispersed phase solutions by dissolving a biodegradable polymer and a drug having different compositions in an organic solvent (step 1);
forming two or more types of dispersed phase emulsions by injecting the two or more types of dispersed phases prepared in the step 1 into an aqueous solution (continuous phase) including a surfactant (step 2);
extracting and evaporating the organic solvent from the dispersed phase emulsion manufactured in the step 2 toward the continuous phase to form a microsphere (step 3); and
recovering the microsphere (step 4) after the step 3.

Specifically, a W/O/W emulsion method of manufacturing a microsphere blending in a single manufacturing process may manufacture by including the following steps 1 to 4 as an example.

Preparing an aqueous phase (W1 phase) by dissolving a drug in an aqueous solution, preparing two or more types of oil phases (O phase) by dissolving a biodegradable polymer having different compositions in an organic solvent, and separately preparing a dispersed phase solution by mixing the aqueous phase (W1 phase) and the two or more types of oil phases (O phase) (step 1);
forming two or more types of dispersed phase emulsions by injecting the two or more dispersed phases prepared in the step 1 into an aqueous solution (W2 phase, continuous phase) including a surfactant (step 2);
extracting and evaporating the organic solvent from the dispersed phase emulsion manufactured in step 2 toward the continuous phase (W2 phase) to form a microsphere (step 3); and
recovering the microsphere after the step 3 (step 4).

### [Embodiment of Invention]

### [Examples]

Hereinafter, the present invention will be described in more detail by the following manufacturing examples. However, the following manufacturing examples are merely illustrative of the present invention, and the content of the present invention is not limited by the following manufacturing examples.

### Examples 1-1 to 1-7 (O/W method): Manufacturing a biodegradable polymer microsphere including cagrilintide and an initial burst inhibiting agent (difference in drug target loading %)

Cagrilintide (manufacturer: Zhejiang Peptides Biotech Co., Ltd, China) was used as a drug, and RG653H and RG753H (manufacturer: Evonik, Germany) were used as a biodegradable polymer at a weight ratio of 1:1 so that a batch size becomes 0.5 g. An oil phase (O phase) in which the drug and the polymer are homogeneously dissolved in a mixed solvent of a dichloromethane (DCM) solvent and a glacial acetic acid co-solvent was prepared as a dispersed phase.

Na₂HPO₄ (Disodium phosphate) as an initial burst inhibiting agent was added to a 0.1 (w/v)% polyvinyl alcohol (viscosity: 4.8 to 5.8 mPa.s) aqueous solution to be 2.0 (w/v)% of final concentration and used as a continuous phase (W phase).

After connecting the continuous phase to an emulsifying apparatus equipped with a porous membrane with a diameter of 40 µm, the prepared dispersed phase was injected into the porous membrane together with the continuous phase. An emulsion in which a biodegradable polymer microdroplet including cagrilintide is dispersed was manufactured, and a suspension was put in a preparation vessel and stirred at a speed of 200 to 300 rpm. A temperature of the preparation vessel was maintained at 25 °C, and after injection of the dispersed phase was completed, the organic solvent was removed while maintaining the suspension at a temperature of 40 °C for 3 hours. Thereafter, the suspension was cooled to a temperature of 25 °C, filtered to remove residual polyvinyl alcohol with tertiary distilled water, and then freeze-dried.

**[Table 1]**

| Example No. | T/L % | Polymer type | Usage amount of polymer (g) | Usage amount of cagrilintide (g) | Usage amount of DCM (g) | Usage amount of glacial acetic acid (g) | Initial burst inhibiting agent and concentration |
|---|---|---|---|---|---|---|---|
| 1-1 | 15 | RG653H:RG753H=1 :1 | 0.425 | 0.075 | 5.2 | 1.425 | 2(w/v)% Na₂HPO₄ |
| 1-2 | 20 | RG653H:RG753H=1 :1 | 0.400 | 0.100 | 4.9 | 1.609 | 2(w/v)% Na₂HPO₄ |
| 1-3 | 25 | RG653H:RG753H=1 :1 | 0.375 | 0.125 | 4.6 | 1.727 | 2(w/v)% Na₂HPO₄ |
| 1-4 | 30 | RG653H:RG753H=1 :1 | 0.350 | 0.150 | 6.7 | 1.702 | 2(w/v)% Na₂HPO₄ |
| 1-5 | 35 | RG653H:RG753H=1 :1 | 0.325 | 0.175 | 6.2 | 1.985 | 2(w/v)% Na₂HPO₄ |
| 1-6 | 40 | RG653H:RG753H=1 :1 | 0.300 | 0.200 | 5.7 | 1.841 | 2(w/v)% Na₂HPO₄ |
| 1-7 | 50 | RG653H:RG753H=1 :1 | 0.250 | 0.250 | 4.8 | 1.734 | 2(w/v)% Na₂HPO₄ |

| | | | | | | | |
|---|---|---|---|---|---|---|---|
| * T/L: Target Loading | | | | | | | |

### Examples 2-1 to 2-4 (O/W method): Manufacturing a biodegradable polymer microsphere including cagrilintide and initial burst inhibiting agent (difference in type of the initial burst inhibiting agent)

Cagrilintide (manufacturer: Zhejiang Peptides Biotech Co., Ltd, China) was used as a drug, and RG653H and RG753H (manufacturer: Evonik, Germany) were used as a biodegradable polymer at a weight ratio of 1:1 so that a batch size becomes 0.5 g (Target Loading 15%). An oil phase (O phase) in which the drug and the polymer are homogeneously dissolved in a mixed solvent of a dichloromethane (DCM) solvent and a glacial acetic acid co-solvent was prepared as a dispersed phase.

K₂HPO₄ (Dipotassium phosphate), Na₂CO₃ (Sodium carbonate), NaHCO₃ (Sodium bicarbonate), or (NH₄)₂HPO₄ (Diammonium phosphate) as an initial burst inhibiting agent was added to 0.1 (w/v)% polyvinyl alcohol (viscosity: 4.8 to 5.8 mPa.s) aqueous solution to be 2.0 (w/v)% of final concentration and used as continuous phase (W phase).

After connecting the continuous phase to an emulsifying apparatus equipped with a porous membrane with a diameter of 40 µm, the prepared dispersed phase was injected into the porous membrane together with the continuous phase. An emulsion in which a biodegradable polymer microdroplet including cagrilintide is dispersed was manufactured, and a suspension was put in a preparation vessel and stirred at a speed of 200 to 300 rpm. A temperature of the preparation vessel was maintained at 25 °C, and after injection of the dispersed phase was completed, the organic solvent was removed while maintaining the suspension at a temperature of 40 °C for 3 hours. Thereafter, the suspension was cooled to a temperature of 25 °C, filtered to remove residual polyvinyl alcohol with tertiary distilled water, and then freeze-dried.

**[Table 2]**

| Example No. | Polymer type | Usage amount of polymer (g) | Usage amount of cagrilintide (g) | Usage amount of DCM (g) | Usage amount of glacial acetic acid (g) | Initial burst inhibiting agent and concentration |
|---|---|---|---|---|---|---|
| 2-1 | RG653H:RG75 3H=1:1 | 0.425 | 0.075 | 5.2 | 1.425 | 2(w/v)% K₂HPO₄ |
| 2-2 | RG653H:RG75 3H=1:1 | 0.425 | 0.075 | 5.2 | 1.425 | 2(w/v)% Na₂CO₃ |
| 2-3 | RG653H:RG75 3H=1:1 | 0.425 | 0.075 | 5.2 | 1.425 | 2(w/v)% NaHCO₃ |
| 2-4 | RG653H:RG75 3H=1:1 | 0.425 | 0.075 | 5.2 | 1.425 | 2(w/v)% (NH₄)₂HPO₄ |

### Examples 3-1 to 3-4 (O/W method): Manufacturing of a biodegradable polymer microsphere including cagrilintide and an initial burst inhibiting agent (difference in type of a polymer)

Cagrilintide (manufacturer: Zhejiang Peptides Biotech Co., Ltd, China) was used as a drug, and RG503H, RG653H, RG753H, or R203H (manufacturer: Evonik, Germany) were used as a biodegradable polymer so that a batch size becomes 0.5 g (Target Loading 15%).

An oil phase (O phase) in which the drug and the polymer are homogeneously dissolved in a mixed solvent of a dichloromethane solvent and a glacial acetic acid co-solvent was prepared as a dispersed phase.

Na₂HPO₄ (Disodium phosphate) as an initial burst inhibiting agent was added to a 0.1 (w/v)% polyvinyl alcohol (viscosity: 4.8~5.8 mPa.s) aqueous solution to be 2.0 (w/v)% of final concentration and used as a continuous phase (W phase).

After connecting the continuous phase to an emulsifying apparatus equipped with a porous membrane with a diameter of 40 µm, the prepared dispersed phase was injected into the porous membrane together with the continuous phase. An emulsion in which a biodegradable polymer microdroplet including cagrilintide is dispersed was manufactured, and a suspension was put in a preparation vessel and stirred at a speed of 200 to 300 rpm. A temperature of the preparation vessel was maintained at 25 °C, and after injection of the dispersed phase was completed, the organic solvent was removed by maintaining the suspension at a temperature of 40 °C for 3 hours. Thereafter, the suspension was cooled to a temperature of 25 °C, filtered to remove residual polyvinyl alcohol with tertiary distilled water, and then freeze-dried.

**[Table 3]**

| Example No. | Polymer type | Usage amount of polymer (g) | Usage amount of cagrilintide (g) | Usage amount of DCM (g) | Usage amount of glacial acetic acid (g) | Initial burst inhibiting agent and concentration |
|---|---|---|---|---|---|---|
| 3-1 | RG503H | 0.425 | 0.075 | 5.2 | 1.425 | 2(w/v)% Na₂HPO₄ |
| 3-2 | RG653H | 0.425 | 0.075 | 5.2 | 1.425 | 2(w/v)% Na₂HPO₄ |
| 3-3 | RG753H | 0.425 | 0.075 | 5.2 | 1.425 | 2(w/v)% Na₂HPO₄ |
| 3-4 | R203H | 0.425 | 0.075 | 5.2 | 1.425 | 2(w/v)% Na₂HPO₄ |

### Examples 4-1 to 4-3 (W/O/W method): Manufacturing a biodegradable polymer microsphere including cagrilintide and an initial burst inhibiting agent (difference in drug target loading %)

As a drug, cagrilintide (manufacturer: Zhejiang Peptides Biotech Co., Ltd, China) was dissolved in 0.665 to 0.770 g of tertiary distilled water (DW) to manufacture a primary aqueous phase (W1 phase). An oil phase (O phase) was manufactured by using RG653H and RG753H (manufacturer: Evonik, Germany) as a biodegradable polymer at a weight ratio of 1:1 by dissolving in 5.2 g of dichloromethane (DCM). An aqueous phase was dissolved in the oil phase using a homogenizer to manufacture a primary W1/O emulsion (dispersed phase).

Na₂HPO₄ (Disodium phosphate) as an initial burst inhibiting agent was added to a 0.1 (w/v)% polyvinyl alcohol (viscosity: 4.8~5.8 mPa.s) aqueous solution to be 2.0 (w/v)% of final concentration and used as a continuous phase (W2 phase).

After connecting the continuous phase to an emulsifying apparatus equipped with a porous membrane with a diameter of 40 µm, the prepared dispersed phase was injected into the porous membrane together with the continuous phase. A secondary W1/O/W2 emulsion in which a biodegradable polymer microdroplet including cagrilintide is dispersed was manufactured, and a suspension was put in a preparation vessel and stirred at a speed of 200 to 300 rpm.

A temperature of the preparation vessel was maintained at 25 °C, and after injection of the dispersed phase was completed, the organic solvent was removed by maintaining the suspension at a temperature of 40 °C for 3 hours. Thereafter, the suspension was cooled to a temperature of 25 °C, filtered to remove residual polyvinyl alcohol with tertiary distilled water, and then freeze-dried.

**[Table 4]**

| Example No. | Polymer type | Usage amount of polymer (g) | Usage amount of cagrilintide (g) | Usage amount of DCM (g) | Weight ratio of DW:DC M | Initial burst inhibiting agent and concentration |
|---|---|---|---|---|---|---|
| 4-1 | RG653H:RG75 3H=1:1 | 0.400 | 0.100 | 7.6 | 1:9.9 | 2(w/v)% Na₂HPO₄ |
| 4-2 | RG653H:RG75 3H=1:1 | 0.375 | 0.125 | 7.1 | 1:10.1 | 2(w/v)% Na₂HPO₄ |
| 4-3 | RG653H:RG75 3H=1:1 | 0.350 | 0.150 | 6.7 | 1:10.0 | 2(w/v)% Na₂HPO₄ |

### Examples 5-1 to 5-6 (O/W method): Manufacturing a biodegradable polymer microsphere including cagrilintide, semaglutide, and an initial burst inhibiting agent (difference in type of a polymer of a co-encapsulation microsphere, difference in drug target loading %)

Cagrilintide (manufacturer: Zhejiang Peptides Biotech Co., Ltd, China) and semaglutide (manufacturer: Chengdu Shengnuo Biopharm Co., Ltd Shengnuo Biopharm Co., Ltd, China) were used as a drug, and RG653H and RG753H (manufacturer: Evonik, Germany) were used at a weight ratio of 1:1, or RG63H or RG753H (manufacturer: Evonik, Germany) was used as a biodegradable polymer and weighed so that a batch size becomes 0.5 g. Dichloromethane (DCM) was used as a solvent for manufacturing a dispersed phase, and glacial acetic acid (acetic acid) was used as a co-solvent to homogeneously dissolve the same.

Na₂HPO₄ (Disodium phosphate) as an initial burst inhibiting agent was added to a 0.1 (w/v)% polyvinyl alcohol (viscosity: 4.8~5.8 mPa.s) aqueous solution to be 2.0 to 2.5 (w/v)% of final concentration and used as a continuous phase (W phase).

After connecting the continuous phase to an emulsifying apparatus equipped with a porous membrane with a diameter of 40 µm, the prepared dispersed phase was injected into the porous membrane together with the continuous phase. An emulsion in which a biodegradable polymer microdroplet including cagrilintide is dispersed was manufactured, and a suspension was put in a preparation vessel and stirred at a speed of 200 to 300 rpm.

A temperature of the preparation vessel was maintained at 25 °C, and after injection of the dispersed phase was completed, the organic solvent was removed by maintaining the suspension at a temperature of 40 °C for 3 hours. Thereafter, the suspension was cooled to a temperature of 25 °C, filtered to remove residual polyvinyl alcohol with tertiary distilled water, and then freeze-dried.

**[Table 5]**

| Example No. | T/L % | Polymer type | Usage amount of polymer (g) | Usage amount of cagrilintide (g) | Usage amount of semaglutide (g) | Usage amount of DCM (g) | Usage amount of glacial acetic acid (g) | Initial burst inhibiting agent and concentration |
|---|---|---|---|---|---|---|---|---|
| 5-1 | 10/10 | RG653H:R G753H=1: 1 | 0.400 | 0.050 | 0.050 | 4.9 | 2.105 | 2 (w/v) % Na₂HP O₄ |
| 5-2 | 10/10 | RG653H | 0.400 | 0.050 | 0.050 | 4.9 | 2.411 | 2 (w/v) % Na₂HP O₄ |
| 5-3 | 10/10 | RG753H | 0.400 | 0.050 | 0.050 | 4.9 | 2.411 | 2 (w/v) % Na₂HP O₄ |
| 5-4 | 12.5/12.5 | RG653H | 0.375 | 0.0625 | 0.0625 | 7.1 | 2.800 | 2 (w/v) % Na₂HP O₄ |
| 5-5 | 12.5/12.5 | RG753H | 0.375 | 0.0625 | 0.0625 | 7.1 | 2.800 | 2 (w/v) % Na₂HP O₄ |
| 5-6 | 15/15 | RG753H | 0.350 | 0.075 | 0.075 | 6.7 | 3.024 | 2.5 (w/v )% Na₂HP O₄ |

| | | | | | | | | |
|---|---|---|---|---|---|---|---|---|
| * T/L: Target Loading (cagrilintide/ semaglutide) | | | | | | | | |

### Examples 6-1 to 6-6 (microsphere blending): Manufacturing a blending of a cagrilintide microsphere and a semaglutide microsphere

After mixing a semaglutide microsphere (A) and a cagrilintide microsphere (B) in a mixing ratio (weight ratio) A:B of 2:1 to 1:2, respectively, a content of each drug in a microsphere blending was confirmed.

**[Table 6]**

| Example | Semaglutide microsphere (A) | Semaglutide content | Cagrilintide microsphere (B) | Cagrilintide content | Mixing ratio (A:B) |
|---|---|---|---|---|---|
| 6-1 | Manufacturing Example 1 | 16.0% | Example 1-2 | 17.35% | 1:1 |
| 6-2 | Manufacturing Example 1 | 16.0% | Example 1-2 | 17.35% | 2:1 |
| 6-3 | Manufacturing Example 1 | 16.0% | Example 1-2 | 17.35% | 1:2 |
| 6-4 | Manufacturing Example 2 | 13.3% | Example 4-1 | 18.3% | 1:1 |
| 6-5 | Manufacturing Example 2 | 13.3% | Example 4-1 | 18.3% | 2:1 |
| 6-6 | Manufacturing Example 2 | 13.3% | Example 4-1 | 18.3% | 1:2 |

The semaglutide microsphere was manufactured through Manufacturing Example 1 and Manufacturing Example 2 below.

### Preparation Example 1 (O/W method): Manufacturing of a polymer microsphere including semaglutide

0.450g of semaglutide freebase (manufacturer: Chengdu Shengnuo Biopharm Co., Ltd, China) was used as a drug and 1.60g RG653H:RG753H=1:1 was used as a biodegradable polymer and weighed so that a batch size becomes 2.0 g. Dichloromethane was used as a solvent for manufacturing a dispersed phase, and glacial acetic acid was used as a co-solvent to prepare an oil phase (O phase) in which the drug and the polymer are homogeneously dissolved as the dispersed phase.

Na₂HPO₄ (Disodium phosphate) as an initial burst inhibiting agent was added to a 0.1 (w/v)% polyvinyl alcohol (viscosity: 4.8~5.8 mPa.s) aqueous solution to be 2.0 to 2.5 (w/v)% of final concentration and used as a continuous phase (W phase).

After connecting the continuous phase to an emulsifying apparatus equipped with a porous membrane with a diameter of 40 µm, the prepared dispersed phase was injected into the porous membrane together with the continuous phase. An emulsion in which a biodegradable polymer microdroplet including semaglutide is dispersed was manufactured, and a suspension was put in a preparation vessel and stirred at a speed of 200 to 300 rpm.

A temperature of the preparation vessel was maintained at 25 °C, and after injection of the dispersed phase was completed, the organic solvent was removed while maintaining the suspension at a temperature of 40 °C for 3 hours. Thereafter, the suspension was cooled to a temperature of 25 °C, filtered to remove residual polyvinyl alcohol with tertiary distilled water, and then freeze-dried.

### Preparation Example 2 (O/W method): Manufacturing a polymer microsphere including semaglutide

0.450g of semaglutide freebase (manufacturer: Chengdu Shengnuo Biopharm Co., Ltd, China) was used as a drug and 2.55g of RG653H:RG753H=1:1 was used as a biodegradable polymer and weighed so that a batch size becomes 3.0 g. Dichloromethane was used as a solvent for manufacturing a dispersed phase, an oil phase (O phase) was prepared by homogeneously dissolving the drug and the polymer as the dispersed phase.

Na₂HPO₄ (Disodium phosphate) as an initial burst inhibiting agent was added to a 0.1 (w/v)% polyvinyl alcohol (viscosity: 4.8~5.8 mPa.s) aqueous solution to be 2.0 to 2.5 (w/v)% of final concentration and used as a continuous phase (W phase).

After connecting the continuous phase to an emulsifying apparatus equipped with a porous membrane with a diameter of 40 µm, the prepared dispersed phase was injected into the porous membrane together with the continuous phase. An emulsion in which a biodegradable polymer microdroplet including semaglutide is dispersed was manufactured, and a suspension was put in a preparation vessel and stirred at a speed of 200 to 300 rpm.

A temperature of the preparation vessel was maintained at 25 °C, and after injection of the dispersed phase was completed, the organic solvent was removed while maintaining the suspension at a temperature of 40 °C for 3 hours. Thereafter, the suspension was cooled to a temperature of 25 °C, filtered to remove residual polyvinyl alcohol with tertiary distilled water, and then freeze-dried.

### Comparative Example 1 (O/W method): Manufacturing of a biodegradable polymer microsphere including cagrilintide without including an initial burst inhibiting agent

0.075 g of cagrilintide (manufacturer: Zhejiang Peptides Biotech Co., Ltd, China) was used as a drug, and 0.425 g of RG653H and RG753H (manufacturer: Evonik, Germany) were used as a biodegradable polymer at a weight ratio of 1:1 so that a batch size becomes 0.5 g. Dichloromethane (DCM) was used as a solvent for manufacturing a dispersed phase, and glacial acetic acid (acetic acid) was used as a co-solvent to homogeneously dissolve the same. 2,000ml of 0.1(w/v)% polyvinyl alcohol (viscosity: 4.8~5.8mPa.s) aqueous solution was used as a continuous phase. After connecting the continuous phase to an emulsifying apparatus equipped with a porous membrane with a diameter of 40 µm, the prepared dispersed phase was injected into the porous membrane together with the continuous phase. An emulsion in which a biodegradable polymer microdroplet including cagrilintide is dispersed was manufactured, and a suspension was put in a preparation vessel and stirred at a speed of 200 to 300 rpm.

A temperature of the preparation vessel was maintained at 25 °C, and after injection of the dispersed phase was completed, the organic solvent was removed while maintaining the suspension at a temperature of 40 °C for 3 hours. Thereafter, the suspension was cooled to a temperature of 25 °C, filtered to remove residual polyvinyl alcohol with tertiary distilled water, and then freeze-dried.

**[Table 7 ]**

| Comparative Example No. | Polymer type | Usage amount of polymer (g) | Usage amount of cagrilintide (g) | Usage amount of DCM (g) | Usage amount of glacial acetic acid (g) |
|---|---|---|---|---|---|
| Comparative Example 1 | RG653H:RG753H= 1:1 | 0.425 | 0.075 | 5.2 | 1.433 |

### Comparative Example 2 (W/O/W method): Manufacturing a biodegradable polymer microsphere including cagrilintide without including an initial burst inhibiting agent

As a drug, 0.075 g of cagrilintide (manufacturer: Zhejiang Peptides Biotech Co., Ltd, China) was dissolved in 0.675 g of tertiary distilled water (DW) to manufacture a primary aqueous phase (W1 phase). An oil phase (O phase) was manufactured by mixing RG653H and RG753H (manufacturer: Evonik, Germany) as a biodegradable polymer at a weight ratio of 1:1 to become 0.425 g and dissolving the same in 3.7 g of dichloromethane (DCM). An aqueous phase was dissolved in an organic phase using a homogenizer to manufacture a primary W1/O emulsion (dispersed phase). 2,000 ml of 0.1 (w/v)% polyvinyl alcohol (viscosity: 4.8-5.8 mPa·s) aqueous solution was used as a continuous phase. After connecting the continuous phase to an emulsifying apparatus equipped with a porous membrane with a diameter of 40 µm, the prepared dispersed phase was injected into the porous membrane together with the continuous phase. A secondary W1/O/W2 emulsion in which a biodegradable polymer microdroplet including cagrilintide is dispersed was manufactured, and a suspension was put in a preparation vessel and stirred at a speed of 200 to 300 rpm.

A temperature of the preparation vessel was maintained at 25 °C, and after injection of the dispersed phase was completed, the organic solvent was removed while maintaining the suspension at a temperature of 40 °C for 3 hours. Thereafter, the suspension was cooled to a temperature of 25 °C, filtered to remove residual polyvinyl alcohol with tertiary distilled water, and then freeze-dried.

**[Table 8]**

| Comparative Example No. | Polymer type | Usage amount of polymer (g) | Usage amount of cagrilintide (g) | Usage amount of DCM (g) | Weight ratio of DW:DCM |
|---|---|---|---|---|---|
| Comparative Example 2 | RG653H:RG753H= 1:1 | 0.425 | 0.075 | 3.7 | 1:5.48 |

### Experimental Example 1. Measuring an encapsulation amount of cagrilintide in a microsphere

In order to measure a drug content of the microsphere manufactured in Examples, 10 mg of the microsphere was completely dissolved in 4.0 mL of acetonitrile, and then was extracted by adding 6.0 mL of 0.1% (w/w) trifluoroacetic acid aqueous solution. A liquid filtered using a 0.45 um filter was used as a test solution. 20 uL of the test solution was injected into HPLC and measured at a detection wavelength of 214 nm. A column used in the present Experimental Example is ZORBAX 300SB-C18, 5 µm, 4.6 x 150 mm, and acetonitrile including 0.1% (w/w) trifluoroacetic acid and a 0.1% (w/w) trifluoroacetic acid aqueous solution was used by mixing in a ratio of 40:60 (v/v).

**[Table 9]**

| | **Cagrilintide encapsulation rate (%)** | **Cagrilintide content (wt%)** | **Semaglutide encapsulation rate (%)** | **Semaglutide content (wt%)** |
|---|---|---|---|---|
| Example 1-1 | 92.10 | 13.81 | N.A | N.A |
| Example 1-2 | 86.70 | 17.35 | N.A | N.A |
| Example 1-3 | 74.20 | 18.56 | N.A | N.A |
| Example 1-4 | 84.85 | 25.46 | N.A | N.A |
| Example 1-5 | 89.21 | 31.22 | N.A | N.A |
| Example 2-1 | 101.97 | 15.30 | N.A | N.A |
| Example 2-2 | 103.40 | 15.51 | N.A | N.A |
| Example 2-3 | 103.04 | 15.46 | N.A | N.A |
| Example 2-4 | 66.58 | 9.99 | N.A | N.A |
| Example 3-1 | 90.90 | 13.64 | N.A | N.A |
| Example 3-2 | 95.00 | 14.25 | N.A | N.A |
| Example 3-3 | 99.49 | 14.92 | N.A | N.A |
| Example 3-4 | 98.40 | 14.76 | N.A | N.A |
| Example 4-1 | 91.55 | 18.31 | N.A | N.A |
| Example 4-2 | 94.18 | 23.55 | N.A | N.A |
| Example 4-3 | 91.14 | 27.34 | N.A | N.A |
| Example 5-1 | 92.80 | 9.28 | 87.51 | 8.75 |
| Example 5-2 | 85.22 | 8.52 | 85.75 | 8.58 |
| Example 5-3 | 86.82 | 8.68 | 87.02 | 8.70 |
| Example 5-4 | 81.32 | 10.17 | 83.98 | 10.50 |
| Example 5-5 | 88.43 | 11.05 | 91.59 | 11.45 |
| Example 5-6 | 77.28 | 11.59 | 84.26 | 12.64 |
| Comparative Example 1 | 106.30 | 15.94 | N.A | N.A |
| Comparative Example 2 | 99.50 | 14.92 | N.A | N.A |

As shown in Table 9,
when viewing a result of Examples 1-1 to 1-5 in which a T/L content of a drug differs in the O/W method, it may be confirmed that the encapsulation rate is maintained at 70% or more up to T/L 35% (Example 1-5),
when viewing a result of Examples 4-1 to 4-3 in which the T/L content of the drug differs in the W/O/W method, it may be confirmed that the encapsulation rate is maintained at 90% or more up to T/L 30% (Example 4-3),
when viewing a result of Examples 2-1 to 2-4 in which a type of an initial burst inhibiting agent differs in the O/W method, it may be confirmed that in a case of Example 2-4 using (NH₄)₂HPO₄ (Diammonium phosphate), the encapsulation rate is rapidly reduced to less than 70% but remaining three types of the initial burst inhibiting agents does not to affect the encapsulation rate,
when viewing a result of Examples 3-1 to 3-4 in which a type of a polymer differs in the O/W method, the type of the polymer did not show any particular effect on the encapsulation rate,
when viewing a result of co-encapsulation microsphere Examples 5-1 to 5-3 in which the type of the polymer differs in the O/W method, the type of the polymer did not show any particular effect on the encapsulation rate, and
when viewing a result of co-encapsulation microsphere Examples 5-3, 5-5, and 5-6 in which a T/L content of a drug differs in the O/W method, it may be confirmed that as the T/L content of the drug increases, the encapsulation rate decreases, but it is still maintained at 70% or more.

### Experimental Example 2. Measuring initial burst of a drug of a cagrilintide microsphere in vitro

In order to confirm an initial burst of a drug from the microsphere manufactured in Examples and Comparative Examples, the following experiment was performed. 10 mg of the microsphere was put in an HDPE wide-mouth bottle, filled with 50 mL of a release test solution, and stored in a 37 °C incubator. After 24 hours, a supernatant obtained by taking and centrifuging 1 mL of the test solution was analyzed for cagrilintide and semaglutide content and release rate using HPLC under the same analysis conditions as in Experimental Example 1. The release test solution used for this measurement was a pH 7.4 aqueous solution including 0.1% polysorbate 20.

**[Table 10]**

| | **In vitro initial burst rate (%, 1-day)** | |
|---|---|---|
| | **Cagrilintide** | **Semaglutide** |
| Example 1-1 | 0.96 | N.A |
| Example 1-2 | 1.54 | N.A |
| Example 1-3 | 4.73 | N.A |
| Example 1-4 | 3.99 | N.A |
| Example 1-5 | 5.81 | N.A |
| Example 1-6 | 6.92 | N.A |
| Example 1-7 | 7.65 | N.A |
| Example 2-1 | 0.30 | N.A |
| Example 2-2 | 0.25 | N.A |
| Example 2-3 | 0.24 | N.A |
| Example 2-4 | 0.51 | N.A |
| Example 3-1 | 0.18 | N.A |
| Example 3-2 | 0.36 | N.A |
| Example 3-3 | 0.42 | N.A |
| Example 3-4 | 0.85 | N.A |
| Example 4-1 | 3.40 | N.A |
| Example 4-2 | 3.44 | N.A |
| Example 5-1 | 2.68 | 2.82 |
| Example 5-2 | 1.99 | 1.48 |
| Example 5-3 | 2.33 | 1.96 |
| Example 5-4 | 5.02 | 5.12 |
| Example 5-5 | 4.99 | 5.23 |
| Example 5-6 | 7.09 | 7.41 |

As shown in Table 10, it was confirmed that as the initial burst inhibiting agent was included in the continuous phase during the manufacturing of the microsphere, the initial burst of the drug from the microsphere including the cagrilintide alone and the co-encapsulation microsphere including the cagrilintide and the semaglutide was significantly reduced.

### Experimental Example 3. Morphological Analysis Through an Electron Microscope

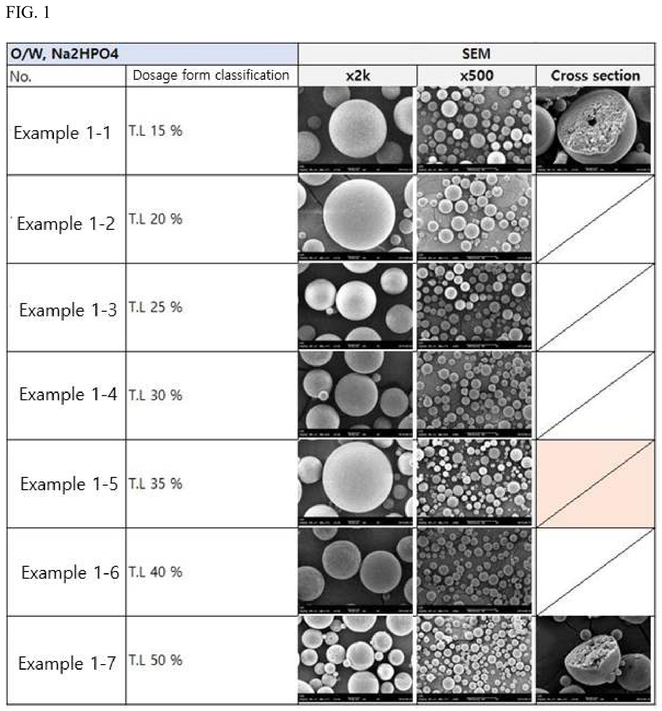

Morphological features of the microsphere according to the present invention were analyzed using an electron microscope. The experimental procedure is as follows. 5 mg of the microsphere manufactured in Examples and Manufacturing Examples was placed on an aluminum stub on which a carbon tape is attached and coated with platinum using an ION-COATER (COXEM, Korea). The aluminum stub was equipped on a scanning electron microscope (COXEM EM-30, Korea) and the morphological features of the microsphere were observed at an acceleration voltage of 10 kV or more and a result is shown in FIG. 1 to FIG. 6.
FIG. 1 is a scanning electron microscope photograph confirming morphological features of a microsphere manufactured in Examples 1-1 to 1-7.

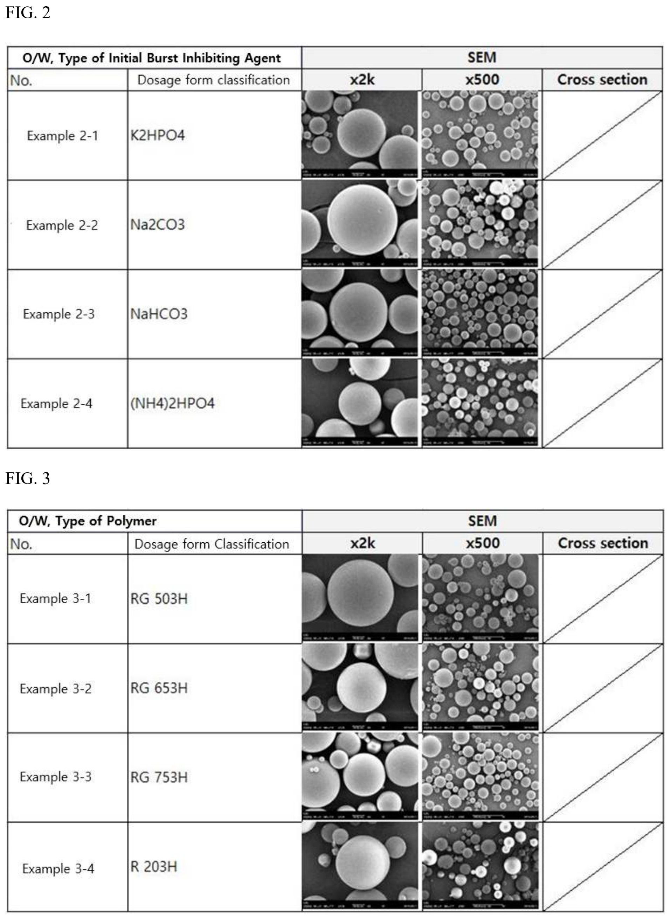

FIG. 2 is a scanning electron microscope photograph confirming morphological features of a microsphere manufactured in Examples 2-1 to 2-4.
FIG. 3 is a scanning electron microscope photograph confirming morphological features of a microsphere manufactured in Examples 3-1 to 3-4.

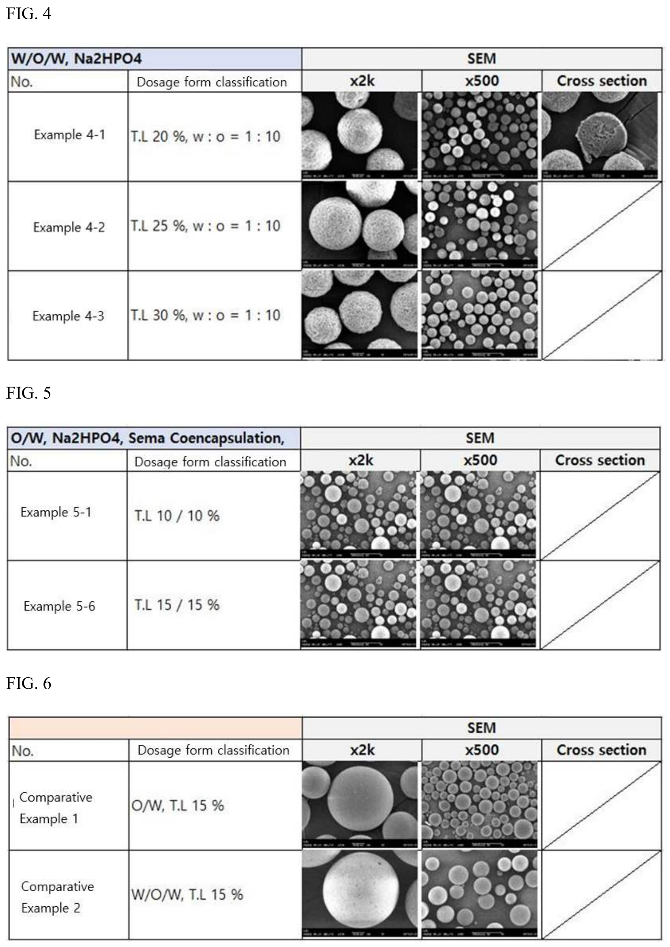

FIG. 4 is a scanning electron microscope photograph confirming morphological features of a microsphere manufactured in Examples 4-1 to 4-3.
FIG. 5 is a scanning electron microscope photograph confirming morphological features of a co-encapsulation microsphere manufactured in Examples 5-1 and 5-6.
FIG. 6 is a scanning electron microscope photograph confirming morphological features of a microsphere manufactured in Comparative Examples 1 and 2.

As shown in FIG. 1 to 6, it was confirmed that all microspheres manufactured by the O/W method had a uniform and smooth surface, and it was confirmed that the microspheres manufactured by the W/O/W method had a uniform surface, but some surfaces were not smooth, but were of good quality.

### Experimental Example 4. Evaluation of in-vivo pharmacokinetics using rats

In order to evaluate an in-vivo drug release pattern of the microsphere according to an embodiment of the present invention, a concentration of cagrilintide in blood was measured after administration to rats.

The microsphere was measured to be 1.2 mg/head (4.0 mg/kg) or 3.6 mg/head (12.0 mg/kg) as the cagrilintide, dispersed in 0.5 mL of a suspension, and then subcutaneously injected into Sprague-Dawley (SD) rats (300 g). 0.5 mL of blood was collected at prescheduled times and the concentration of the cagrilintide in blood was measured using HPLC. Here, in a case of a co-encapsulation microsphere (Examples 5-1 and 5-6), a blood concentration of the cagrilintide excluding the semaglutide was analyzed.

**[Table 11]**

| Experiment no. | G4 | G5 | G6 | G7 | G18 |
|---|---|---|---|---|---|
| Example no. | 1-1 | 1-2 | 1-3 | 5-1 | 5-6 |
| T/L %(Cagrilintide / semaglutide) | 15 | 20 | 25 | 10/10 | 15/15 |
| Dose(mg/head) | 3.6 | 3.6 | 3.6 | 3.6 | 1.2 |
| AUCnorm(ng*day/mL)/(mg/head) | 208.6 | 651.2 | 1796.4 | 4898.8 | 5486.7 |
| Cmax(ng/ml) | 41.7 | 173.4 | 690.1 | 1512.8 | 407.23 |

As shown in Table 11, it was confirmed that a bioavailability of the co-encapsulation microsphere with the cagrilintide and the semaglutide (Examples 5-1 and 5-6) tends to be significantly improved compared to the microsphere with the cagrilintide alone (Examples 1-1 to 1-3).

### Experimental Example 5. Experiment of pharmacokinetics of a single-dose subcutaneous administration using SD rats

In order to evaluate a potential of the microsphere according to the present invention as a sustained-release therapeutic agent, the concentration of the cagrilintide in rat blood was measured by the following method.

Specifically, the microsphere was measured so that a dosage of the cagrilintide becomes 1.2 mg/head (API raw material solution, Example 5-6) or 3.6 mg/head (Comparative Examples 1 to 2, Examples 1-1 to 1-3, Example 5-1), dispersed in 0.5 mL of a suspension, and then subcutaneously injected into SD rats. 0.25 to 0.5 mL of blood was collected at prescheduled times and the concentration of the cagrilintide in blood was measured using HPLC.

**[Table 12]**

| | Time(day)-Concentration (ng/mL) | | | | | | | | | |
|---|---|---|---|---|---|---|---|---|---|---|
| | 0 | 0.042 | 0.250 | 1 | 4 | 7 | 10 | 14 | 17 | 21 |
| API raw material solution | 0.0% | 0.2% | 4.5% | 35.2% | 91.1% | 100.0% | | | | |
| Comparative Example 2 | 0.0% | 0.1% | 2.2% | 19.6% | 71.1% | 78.6% | 80.8% | 82.7% | 84.5% | 87.6% |
| Comparative Example 1 | 0.0% | 0.0% | 0.8% | 11.4% | 51.2% | 58.9% | 61.4% | 63.1% | 63.8% | 65.1% |
| Example 1-1 | 0.0% | 0.0% | 0.2% | 2.9% | 13.8% | 18.1% | 21.6% | 28.7% | 33.2% | 37.4% |
| Example 1-2 | 0.0% | 0.0% | 0.2% | 3.7% | 19.1% | 26.7% | 33.0% | 42.3% | 48.6% | 54.4% |
| Example 1-3 | 0.0% | 0.0% | 0.2% | 4.7% | 27.5% | 37.3% | 41.9% | 48.2% | 52.6% | 56.5% |
| Example 5-1 | 0.0% | 0.0% | 0.1% | 0.8% | 3.3% | 4.4% | 6.1% | 12.4% | 27.4% | 55.8% |
| Example 5-6 | 0.0% | 0.0% | 0.6% | 6.2% | 28.8% | 38.5% | 45.1% | 52.9% | 58.3% | 66.2% |

| | 28 | 35 | 42 | 49 | 56 | 63 | 70 | 77 | 84 | |
|---|---|---|---|---|---|---|---|---|---|---|
| API raw material solution | | | | | | | | | | |
| Comparative Example 2 | 91.8% | 94.6% | 96.2% | 97.1% | 98.1% | 99.1% | 99.7% | 100.0% | | |
| Comparative Example 1 | 70.2% | 82.1% | 91.7% | 94.2% | 96.5% | 98.9% | 100.0% | | | |
| Example 1-1 | 43.6% | 53.9% | 65.7% | 74.7% | 86.6% | 96.8% | 100.0% | | | |
| Example 1-2 | 59.8% | 64.6% | 69.7% | 74.6% | 80.8% | 88.3% | 94.5% | 97.9% | 100.0% | |
| Example 1-3 | 61.3% | 70.5% | 80.2% | 86.8% | 93.6% | 97.4% | 98.8% | 99.6% | 100.0% | |
| Example 5-1 | 79.8% | 83.5% | 88.6% | 93.4% | 97.0% | 99.2% | 99.9% | 100.0% | | |
| Example 5-6 | 78.7% | 85.3% | 89.9% | 96.0% | 100.0% | | | | | |

As shown in Table 12,
The microsphere of Comparative Examples 1 and 2 in which the initial burst inhibiting agent was not used showed an initial burst with a cumulative blood concentration exceeding 10% on day 1, whereas all of Examples 1-1 to 1-3, Example 5-1, and Example 5-6 showed a cumulative blood concentration of 10% or less on day 1, and an excellent sustained-release profile was confirmed with a continuous release for a short period of 56 days and a long period of 84 days.

### Experimental Example 6. Measuring a of residual content of an initial burst inhibiting agent in a microsphere

In order to measure a content of an initial burst inhibiting agent in the microsphere manufactured in Examples, a residual content of sodium (Na) and phosphorus (P) in the microsphere was measured.

Specifically, 300 mg of the microsphere was mixed with 6 mL of a nitric acid aqueous solution and 3 mL of hydrogen peroxide mixed with ultrapure water in a 1:1 ratio, heated at 100 °C or higher, and an acid was added until a gas generated during the dissolution process changed from yellow to white. A sample obtained through this was weighed, dissolved in ultrapure water, and then injected into an inductively coupled plasma optical emission spectrometer (ICP-OES) (Thermo Scientific Co., iCAP 6300 Duo, UK) and measured at a detection wavelength of 598.5 nm.

**[Table 13]**

| | Example 3-4 | Example 1-1 | Example 5-6 | Example 2-1 |
|---|---|---|---|---|
| Initial burst inhibiting agent | 2(w/v)% Na₂HPO₄ | 2(w/v)% Na₂HPO₄ | 2.5(w/v)% Na₂HPO₄ | 2(w/v)% K₂HPO₄ |
| Polymer | R203H | RG 653H:RG 753H | RG 753H | RG 653H:RG 753H |
| Residual Sodium (mg/kg) | 399 | 105 | 171 | - |
| Residual Phosphorus (mg/kg) | 81 | 33 | 54 | 41 |

As shown in Table 13, a content of residual sodium and phosphorus according to the initial burst inhibiting agent included in the continuous phase differs depending on a manufacturing condition of the microsphere, and a content of the initial burst inhibiting agent remaining in the microsphere calculated from this was confirmed to be 50 ppm to 500 ppm based on sodium (Na) and 10 to 100 ppm based on phosphorus (P).

## Claims

1. A pharmaceutical composition for preventing and treating diabetes, type 2 diabetes, beta-cell dysfunction, obesity, non-alcoholic steatohepatitis, or Alzheimer's disease, the pharmaceutical composition comprising sustained-release microspheres containing:
as an active ingredient, (i) cagrilintide or a pharmaceutically acceptable salt thereof, or (ii) cagrilintide or a pharmaceutically acceptable salt thereof and semaglutide or a pharmaceutically acceptable salt thereof;
an initial burst inhibiting agent; and
a biodegradable polymer,
wherein the cagrilintide or the pharmaceutically acceptable salt thereof of the (i) is included in an amount of 8 wt.% or more as cagrilintide based on the total weight of the microsphere,
the cagrilintide or the pharmaceutically acceptable salt thereof of the (ii) is included in an amount of 4 wt.% or more as the cagrilintide based on the total weight of the microsphere, and
a content of the initial burst inhibiting agent is included in 10 to 2,000 ppm.

2. A pharmaceutical composition for preventing and treating diabetes, type 2 diabetes, beta-cell dysfunction, obesity, non-alcoholic steatohepatitis, or Alzheimer's disease, the pharmaceutical composition comprising:
a combination of sustained-release microspheres consisting of cagrilintide or a pharmaceutically acceptable salt thereof as an active ingredient, an initial burst inhibiting agent, and a biodegradable polymer; and sustained-release microsphere including semaglutide or a pharmaceutically acceptable salt thereof as an active ingredient, an initial burst inhibiting agent, and a biodegradable polymer,
wherein the cagrilintide or the pharmaceutically acceptable salt thereof is included in an amount of 8 wt.% or more as the cagrilintide based on the total weight of a microsphere, and
a content of the initial burst inhibiting agent is included in 10 to 2,000 ppm.

3. The pharmaceutical composition of claim 1 or claim 2, wherein a release rate of the cagrilintide or the pharmaceutically acceptable salt thereof in the microsphere is less than 20% within 24 hours when administered in vivo.

4. The pharmaceutical composition of claim 1 or claim 2, wherein a release rate of the cagrilintide or the pharmaceutically acceptable salt thereof in the microsphere is less than 15% within 24 hours when administered in vivo.

5. The pharmaceutical composition of claim 1 or claim 2, wherein a release rate of the cagrilintide or the pharmaceutically acceptable salt thereof in the microsphere is less than 10% within 24 hours when administered in vivo.

6. The pharmaceutical composition of claim 1 or claim 2, wherein the biodegradable polymer is at least one selected from a group consisting of:
a polymer selected from a group consisting of polylactide (PLA), polyglycolide (PGA), polylactide-co-glycolide (PLGA) that is a copolymer of lactide and glycolide, polydioxanone, polycaprolactone (PCL), polylactide-co-glycolide-co-caprolactone (PLGC), polylactide-co-hydroxymethyl glycolide (PLGMGA), polyalkylcarbonate, polytrimethylenecarbonate (PTMC), polylactide-co-trimethylenecarbonate (PLTMC), polyhydroxybutyric acid (PHB), polyhydroxybutyrate-co-hydroxyvalerate (PHBV), polyorthoester, polyanhydride, polyanhydride-co-imide, polypropylene fumarate, pseudo polyaminoacid, polyalkyl cyanoacrylate, polyphosphazene, polyphosphoester, polysaccharide, and poly(butylene succinate lactide) (PBSLA); a copolymer or a simple mixture of two or more types of the selected polymer; a copolymer with the polymer and polyethylenglycol (PEG); a polymer-sugar complex in which said polymer or said copolymer and sugar are bonded.

7. The pharmaceutical composition of claim 1 or 2, wherein the initial burst inhibiting agent is one or more types of substances selected from phosphate salt, hydroxide salt, phosphide salt, phosphite salt, carbonate salt, chromate salt, dichromate salt, oxide, oxalate salt, silicate salt, sulfate salt, sulfide salt, sulfite salt, tartrate salt, tetraborate salt, thiosulfate salt, arsenate salt, arsenite salt, citrate salt, ferricyanide salt, and nitride salt of an alkali metal, an alkaline-earth metal, or ammonium.

8. The pharmaceutical composition of claim 1 or claim 2, wherein an average particle size of the microsphere including the cagrilintide or the pharmaceutically acceptable salt thereof and the initial burst inhibiting agent is 5 µm to 100 µm.

9. The pharmaceutical composition of claim 1 or claim 2, wherein a weight of the microsphere including the cagrilintide or the pharmaceutically acceptable salt thereof and the initial burst inhibiting agent is 20 mg to 1000 mg.

10. The pharmaceutical composition of claim 1 or claim 2, wherein an intrinsic viscosity of the biodegradable polymer is 0.16 dL/g to 1.7 dL/g.

11. The pharmaceutical composition of claim 1 or claim 2, wherein the microsphere further includes one or more types of release control agents selected from a group consisting of butyric acid, valeric acid, caproic acid, enantic acid, caprylic acid, pelargonic acid, capric acid, undecylic acid, lauric acid, tridecylic acid, myristic acid, pentadecylic acid, palmitic acid, heptadecylic acid, stearic acid, nonadecylic acid, behenic acid, arachidic acid, isocrotonic acid, oleic acid, elaidic acid, sorbic acid, linoleic acid, arachidonic acid, benzoic acid, hydroxynaphthoic acid, napadisylic acid, naphthalenesulfonic acid, and pamoic acid.

12. The pharmaceutical composition of claim 1,
wherein the semaglutide or the pharmaceutically acceptable salt thereof of the (ii) is included in an amount of 4 wt.% or more as the semaglutide based on the total weight of the microsphere.

13. The pharmaceutical composition of claim 2,
wherein the semaglutide or the pharmaceutically acceptable salt thereof is included in an amount of 8 wt.% or more as the semaglutide based on the total weight of the microsphere.

14. A method of manufacturing a microsphere with an oil-in-water (O/W) emulsion including an active ingredient, an initial burst inhibiting agent, and a biodegradable polymer, the method comprising:
(a) preparing an oil phase (O phase) as a dispersed phase obtained by dissolving (i) cagrilintide or a pharmaceutically acceptable salt thereof, or (ii) cagrilintide or a pharmaceutically acceptable salt thereof and semaglutide or a pharmaceutically acceptable salt thereof as an active ingredient, and one or more of biodegradable polymers in an organic solvent;
(b) preparing a continuous phase (W phase) by adding an initial burst inhibiting agent to an aqueous solution including a surfactant, and then adding the dispersed phase obtained in the step (a) to form an emulsion state of the dispersed phase;
(c) extracting the organic solvent from the dispersed phase in the emulsion state prepared in the step (b) to the continuous phase (W phase) and evaporating the organic solvent to form microspheres; and
(d) recovering the microsphere.

15. A method of preparing a microsphere with a water-in-oil-in-water (W/O/W) emulsion including an active ingredient, an initial burst inhibiting agent, and a biodegradable polymer, the method comprising:
(a') preparing a primary aqueous phase (W1 phase) by dissolving (i) cagrilintide or a pharmaceutically acceptable salt thereof, or (ii) cagrilintide or a pharmaceutically acceptable salt thereof and semaglutide or a pharmaceutically acceptable salt thereof as an active ingredient in an aqueous solution,
and preparing an oil phase (O phase) by dissolving one or more of biodegradable polymers in an organic solvent to prepare a primary W1/O emulsion as a dispersed phase in which the aqueous phase (W1 phase) and the oil phase (O phase) are mixed;
(b') preparing a continuous phase (W2 phase) by adding an initial burst inhibiting agent to an aqueous solution including a surfactant, and then adding the dispersed phase obtained in the step (a') to prepare a dispersed phase in a secondary W1/O/W2 emulsion state;
(c') extracting an organic solvent from the dispersed phase in the secondary W1/O/W2 emulsion state of the step (b') to the continuous phase (W2 phase) and/or evaporating the organic solvent to form a microsphere; and
(d') recovering the microsphere.

16. The method of any one of claim 14 and claim 15, wherein the biodegradable polymer is at least one selected from a group consisting of: a polymer selected from a group consisting of polylactide (PLA), polyglycolide (PGA), polylactide-co-glycolide (PLGA), polydioxanone, polycaprolactone (PCL), polylactide-co-glycolide-co-caprolactone (PLGC), polylactide-co-hydroxymethyl glycolide (PLGMGA), polyalkylcarbonate, polytrimethylenecarbonate (PTMC), polylactide-co-trimethylenecarbonate (PLTMC), polyhydroxybutyric acid (PHB), polyhydroxybutyrate-co-hydroxyvalerate (PHBV), polyorthoester, polyanhydride, polyanhydride-co-imide, polypropylene fumarate, pseudo polyaminoacid, polyalkyl cyanoacrylate, polyphosphazene, polyphosphoester, polysaccharide, and poly(butylene succinate lactide) (PBSLA); a copolymer or a simple mixture of two or more types of the selected polymer; a copolymer with the polymer and polyethylenglycol (PEG); and a polymer-sugar complex in which the polymer or the copolymer and sugar are bonded.

17. The method of any one of claim 14 and claim 15, wherein the dispersed phase of the step (a) or (a') further includes one or more types of release control agents selected from a group consisting of butyric acid, valeric acid, caproic acid, enantic acid, caprylic acid, pelargonic acid, capric acid, undecylic acid, lauric acid, tridecylic acid, myristic acid, pentadecylic acid, palmitic acid, heptadecylic acid, stearic acid, nonadecylic acid, arachidic acid, isocrotonic acid, oleic acid, elaidic acid, sorbic acid, linoleic acid, arachidonic acid, hydroxynaphthoic acid, napadisylic acid, and pamoic acid.

18. The method of any one of claim 14 and claim 15, wherein the organic solvent in the step (a) or (a') is one or more types of organic solvents selected from a group consisting of dichloromethane, chloroform, ethyl acetate, methyl ethyl ketone, acetone, acetonitrile, dimethyl sulfoxide, dimethyl formamide, n-methyl pyrrolidone, acetic acid, methyl alcohol, ethyl alcohol, propyl alcohol, and benzyl alcohol.

19. The method of any one of claim 14 and claim 15, wherein the surfactant in the step (b) or (b') is polyvinyl alcohol.

20. The method of any one of claim 14 and claim 15, wherein a continuous phase of the step (b) or (b') further includes at least one selected from a group consisting of methyl alcohol, ethyl alcohol, propyl alcohol, and ethyl acetate.

21. The method of any one of claim 14 and claim 15, wherein the continuous phase of the step (b) is used by adding the initial burst inhibiting agent to the aqueous solution including the surfactant so that a final concentration becomes 0.1 to 5.0 (w/v)%.

22. The method of any one of claim 14 and claim 15, wherein the initial burst inhibiting agent is at least one selected from a group consisting of phosphate salt of two or more alkali metals, bicarbonate salt of one or more alkali metals, and carbonate salt of one or more alkali metals.

23. The method of any one of claim 14 and claim 15, wherein a pH of the continuous phase in the step (b) or (b') is 7 or more.

24. The method of any one of claim 14 and claim 15, wherein a release rate of the cagrilintide or the pharmaceutically acceptable salt thereof is less than 15% within 24 hours when a prepared sustained-release microsphere is administered in vivo.

25. The method of any one of claim 14 and claim 15, wherein a release rate of the cagrilintide or the pharmaceutically acceptable salt thereof is less than 10% within 24 hours of an in vivo administration of a prepared sustained-release microsphere.
